# EUROPÄISCHE PATENTSCHRIFT

(11) **EP 0 705 828 B1**
(45) Veröffentlichungstag und Bekanntmachung des Hinweises auf die Patenterteilung: **01.12.1999**
(21) Anmeldenummer: 95114839.4
(22) Anmeldetag: 21.09.1995
(51) Int. Cl.: C07D 401/04, A61K 31/395, C07D 491/06, C07D 471/04, C07D 209/44

(54) **Chinolon- und Naphthyridoncarbonsäure-Derivate als antibakterielle Mittel**
Quinolone- and naphthyridone carboxylic acid derivatives as antibacterial agents
Dérivés carboxyliques de quinolones et de naphthyridones comme agents antibactériens

(30) Priorität: 04.10.1994 DE 4435479
(43) Veröffentlichungstag der Anmeldung: 10.04.1996
(73) Patentinhaber: BAYER AG, 51368 Leverkusen (DE)
(72) Erfinder: Petersen, Uwe, Dr., D-51375 Leverkusen (DE); Schenke, Thomas, Dr., D-51469 Bergisch Gladbach (DE); Bremm, Klaus-Dieter, Dr., D-45661 Recklinghausen (DE); Endermann, Rainer, Dr., D-42113 Wuppertal (DE)

(56) Entgegenhaltungen:
- EP-A- 0 429 304
- EP-A- 0 520 240
- WO-A-95/21163
- DE-A- 4 329 600
- JOURNAL OF MEDICINAL CHEMISTRY, Bd. 35, Nr. 25, 1992 WASHINGTON US, Seiten 4745-4750, MARK J. SUTO ET AL. 'Fluoroquinolones: relationships between structural variations, mammalian cell cytotoxicity, and antimicrobial activity.'

## Beschreibung

Die Erfindung betrifft neue Chinolon- und Naphthyridoncarbonsäure-Derivate, die in 7-Stellung durch einen zweifach ungesättigten bicylischen Aminrest substituiert sind, ihre Salze, Verfahren zu ihrer Herstellung sowie diese enthaltende antibakterielle Mittel.

Es sind bereits aus den Patentanmeldungen EP 520 240, DE 42 30 804, DE 43 29 600 (Bayer) sowie JP 4 253 973 (Banyu) Chinoloncarbonsäuren bekannt, die in 7-Stellung durch einen bicyclischen einfach ungesättigten Aminrest substituiert sind. Diese Verbindungen zeichnen sich durch eine hohe antibakterielle Aktivität aus. Sie besitzen aber den Nachteil, daß sie ein hohes gentoxisches Potential aufweisen, was ihren Einsatz als Arzneimittel unmöglich macht. Der Erfindung liegt daher die Aufgabe zugrunde, Verbindungen aufzufinden, die bei hoher antibakterieller Wirksamkeit eine Verringerung der gentoxischen Eigenschaften aufweisen.

Es wurde jetzt gefunden, daß die Verbindungen der Formel (I)

T-Q (I),

in welcher
- Q: einen Rest der Formeln 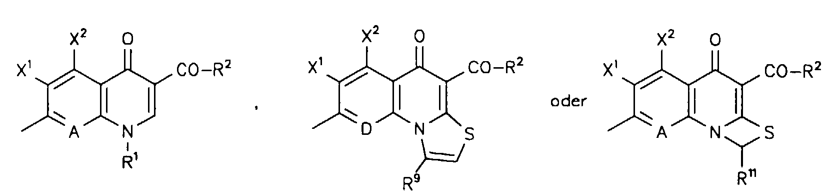 bedeutet, worin
R¹ für gegebenenfalls durch Halogen oder Hydroxy ein- oder zweifach substituiertes Alkyl mit 1 bis 4 Kohlenstoffatomen, Alkenyl mit 2 bis 4 Kohlenstoffatomen, gegebenenfalls durch 1 oder 2 Fluoratome substituiertes Cycloalkyl mit 3 bis 6 Kohlenstoffatomen, Bicyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl,
R² für Hydroxy, gegebenenfalls durch Hydroxy, Methoxy, Amino, Dimethylamino substituiertes Alkoxy mit 1 bis 3 Kohlenstoffatomen, Benzyloxy oder (5-Methyl-2-oxo-1,3-dioxol-4-yl)-methyloxy, Acetoxymethyloxy, Pivaloyloxymethyloxy, 5-Indanyloxy, Phthalidinyl-oxy, 3-Acetoxy-2-oxo-butyloxy, Nitromethyl oder Dialkoxycarbonylmethyl mit 1 bis 2 Kohlenstoffatomen in jedem Alkylteil,
R⁹ für Wasserstoff oder gegebenenfalls durch Methoxy, Hydroxy oder Halogen substituiertes Alkyl mit 1 bis 3 Kohlenstoffatomen,
R¹¹ für Wasserstoff CH₃ oder CH₂F,
X¹ für Halogen oder Nitro,
X² für Wasserstoff Halogen, Amino, Hydroxy, Methoxy, Mercapto, Methyl, Halogenmethyl oder Vinyl,
A für N oder C-R⁷ steht, worin
R⁷ für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*S-CH₂-CH-CH₃, -*CH₂-CH₂-CH-CH₃ oder -*O-CH₂-N-R⁸, wobei das mit * markierte Atom mit dem Kohlenstoffatom von A verknüpft ist und worin
R⁸ Wasserstoff, Methyl oder Formyl bedeutet, bilden kann, und
D für N oder C-R¹⁰ steht, worin
R¹⁰ für Wasserstoff, Halogen, CF₃, OCH₃, OCHF₂ oder CH₃ steht oder auch gemeinsam mit R⁹ eine Brücke der Struktur -*O-CH₂-, -*NH-CH₂-, -*N(CH₃)-CH₂-, -*N(C₂H₅)-CH₂-, -*N(c-C₃H₅)-CH₂- oder -*S-CH₂- bilden kann, wobei das mit * markierte Atom mit dem Kohlenstoffatom von D verknüpft ist, und
T einen Rest der Formel 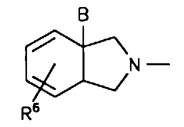 bedeutet, worin
B für (CH₂)ₘ-NR³R⁴ oder (CH₂)ₘ-OR⁵ steht, worin
m für 0 oder 1,
R³ für Wasserstoff Methyl oder Alkoxycarbonyl mit 1 bis 4 Kohlenstoffatomen im Alkylteil,
R⁴ für Wasserstoff oder Methyl und
R⁵ für Wasserstoff oder Methyl und
R⁶ für Wasserstoff oder Methyl steht,
und deren pharmazeutisch verwendbaren Hydrate und Säureadditionssalze sowie die Alkali-, Erdalkali-, Silber- und Guanidiniumsalze der zugrundeliegenden Carbonsäuren bei guter Verträglichkeit eine hohe antibakterielle Wirkung insbesondere gegenüber grampositiven Bakterien aufweisen.

Bevorzugt sind die Verbindungen der Formel (I),
in welcher
- Q: einen Rest der Formeln 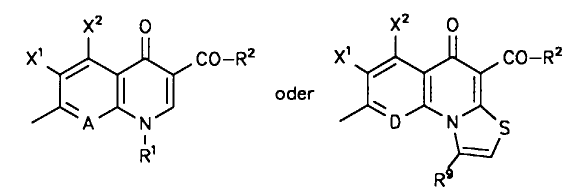
bedeutet, worin
- R¹: für gegebenenfalls durch Halogen ein- oder zweifach substituiertes Alkyl mit 1 bis 4 Kohlenstoffatomen, Alkenyl mit 2 bis 3 Kohlenstoffatomen, gegebenenfalls durch 1 Fluoratom substituiertes Cycloalkyl mit 3 oder 4 Kohlenstoffatomen, Bicyclo[1.1.1]pent-1-yl, 1,1-Dimethylpropargyl, 3-Oxetanyl, Methylamino, gegebenenfalls durch Fluor, Amino oder Hydroxy ein- oder zweifach substituiertes Phenyl,
- R²: für Hydroxy, Alkoxy mit 1 bis 2 Kohlenstoffatomen, Benzyloxy oder (5-Methyl-2-oxo-1,3-dioxol-4-yl)-methoxy,
- R⁹: für Wasserstoff oder gegebenenfalls durch Fluor ein- bis dreifach substituiertes Alkyl mit 1 bis 2 Kohlenstoffatomen,
- X¹: für Fluor oder Chlor,
- X²: für Wasserstoff, Halogen, Amino, Methyl, Trifluormethyl oder Vinyl,
- A: für N oder C-R⁷ steht, worin
R⁷ für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*CH₂-CH₂-CH-CH₃ oder -*O-CH₂-N-R⁸, wobei das mit * markierte Atom mit dem Kohlenstoffatom von A verknüpft ist und worin
R⁸ Wasserstoff oder Methyl bedeutet, bilden kann, und
- D: für N oder C-R¹⁰ steht, worin
R¹⁰ für Wasserstoff, Fluor, Chlor, CF₃, OCH₃ oder CH₃ steht oder auch gemeinsam mit R⁹ eine Brücke der Struktur -*O-CH₂-, -*N(CH₃)-CH₂-, -*N(C₂H₅)-CH₂-, -*N(c-C₃H₅)-CH₂- oder -*S-CH₂- bilden kann, wobei das mit * markierte Atom mit dem Kohlenstoffatom von D verknüpft ist, und
- T: einen Rest der Formel 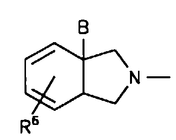 bedeutet, worin
- B: für -NR³R⁴ oder -OH steht, worin
R³ für Wasserstoff oder Methyl,
R⁴ für Wasserstoff oder Methyl und
- R⁶: für Wasserstoff steht
und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I), in welcher
- Q: einen Rest der Formeln 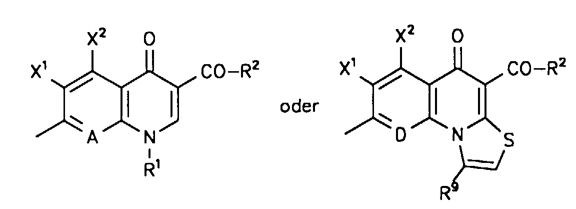
bedeutet, worin
- R¹: für gegebenenfalls durch Fluor ein- oder zweifach substituiertes Alkyl mit 1 bis 4 Kohlenstoffatomen, Vinyl, gegebenenfalls durch 1 Fluoratom substituiertes Cyclopropyl, gegebenenfalls durch Fluor ein- oder zweifach substituiertes Phenyl,
- R²: für Hydroxy, Alkoxy mit 1 bis 2 Kohlenstoffatomen oder (5-Methyl-2-oxo-1,3-dioxol-4-yl)-methoxy,
- R⁹: für Wasserstoff oder gegebenenfalls durch Fluor ein- bis dreifach substituiertes Methyl,
- X¹: für Fluor,
- X²: für Wasserstoff, Fluor, Amino, Methyl oder Vinyl,
- A: für N oder C-R⁷ steht, worin
R⁷ für Wasserstoff, Fluor, Chlor, Brom, CF₃, OCH₃, OCHF₂, CH₃, CN, CH=CH₂ oder C≡CH steht oder auch gemeinsam mit R¹ eine Brücke der Struktur -*O-CH₂-CH-CH₃ oder -*O-CH₂-N-R⁸, wobei das mit * markierte Atom mit dem Kohlenstoffatom von A verknüpft ist und worin
R⁸ Wasserstoff oder Methyl bedeutet, bilden kann, und
- D: für N oder C-R¹⁰ steht, worin
R¹⁰ für Wasserstoff, Fluor, Chlor oder OCH₃ steht oder auch gemeinsam mit R⁹ eine Brücke der Struktur -*O-CH₂-, -*N(CH₃)-CH₂-, -*N(C₂H₅)-CH₂- oder -*S-CH₂- bilden kann, wobei das mit * markierte Atom mit dem Kohlenstoffatom von D verknüpft ist, und
- T: einen Rest der Formel 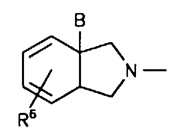 bedeutet, worin
B für NH₂ und
R⁶ für Wasserstoff steht,
und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn man Verbindungen der Fomel (II)

Y-Q (II),

in welcher
- Q: die oben angegebene Bedeutung hat und
- Y: für eine Abgangsgruppe wie Halogen, inbesondere für Fluor oder Chlor steht,
mit Verbindungen der Formel (III) 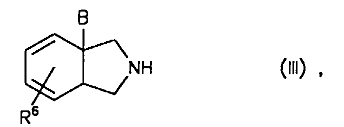 in welcher
B und R⁶ die oben angegebenen Bedeutungen haben,
gegebenenfalls in Gegenwart von Säurefängern umsetzt und gegebenenfalls vorhandene Schutzgruppen abspaltet.

Verwendet man beispielsweise 6,7-Difluor-1-cyclopropyl-1,4-dihydro-8-methoxy-4-oxo-3-chinolincarbonsäure und 1,2,3,7a-Tetrahydro-isoindol-3a-ylamin als Ausgangsstoffe, so kann der Reaktionsverlauf durch das folgende Formelschema wiedergegeben werden: 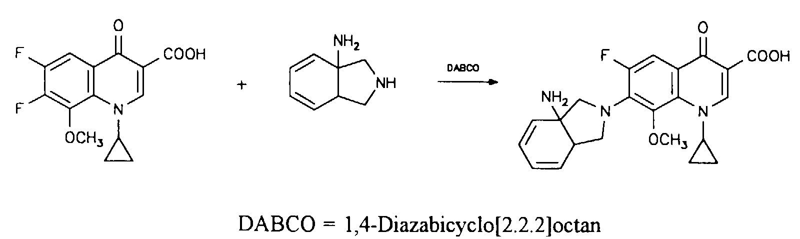 Die als Ausgangsverbindungen verwendeten Verbindungen der Formel (II) sind bekannt oder können nach bekannten Methoden hergestellt werden. Sie können gegebenenfalls sowohl als racemische als auch als enantiomerenreine Verbindungen eingesetzt werden. Bei mangelnder Reaktivität können die Verbindungen der Formel (II) auch als Bor-Chelate eingesetzt werden. Als Beispiele seien genannt:
7-Chlor-1-cyclopropyl-6-fluor-1,4-dihydro-4-oxo-3-chinolincarbonsäure, 1-Cyclopropyl-6,7-difluor-1,4-dihydro-4-oxo-3-chinolincarbonsäure, 6-Chlor-1-cyclopropyl-7,8-difluor-1,4-dihydro-4-oxo-3-chinolincarbonsäure, 8-Chlor-1-cyclopropyl-6,7-difluor-1,4-dihydro-4-oxo-3-chinolincarbonsäure, 1-Cyclopropyl-6,7,8-trifluor-1,4-dihydro-4-oxo-3-chinolincarbonsäure, 5-Brom-1-cyclopropyl-6,7,8-trifluor-1,4-dihydro-4-oxo-3-chinolincarbonsäure, 5-Brom-1-(2,4-difluorphenyl)-6,7,8-trifluor-1,4-dihydro-4-oxo-3-chinolincarbonsäure, 1-Cyclopropyl-6,7-difluor-1,4-dihydro-8-methyl-4-oxo-3-chinolincarbonsäure, 6,7-Difluor-1-ethyl-1,4-dihydro-4-oxo-3-chinolincarbonsäure, 7-Chlor-6-fluor-1-ethyl-1,4-dihydro-4-oxo-3-chinolincarbonsäure, 7-Chlor-6-fluor-1,4-dihydro-1-(2-hydroxyethyl)-4-oxo-3-chinolincarbonsäure, 6,7-Difluor-1-(2-fluorethyl)-1,4-dihydro-4-oxo-3-chinolincarbonsäure, 7-Chlor-6-fluor-1,4-dihydro-1-methoxy-4-oxo-3-chinolincarbonsäure, 7-Chlor-6-fluor-1,4-dihydro-1-methylamino-4-oxo-3-chinolincarbonsäure, 6,7-Difluor-1,4-dihydro-4-oxo-1-phenyl-3-chinolincarbonsäure, 7-Chlor-1-cyclopropyl-6-fluor-1,4-dihydro-4-oxo-1,8-naphthyridin-3-carbonsäure, 7-Chlor-1-cyclopropyl-6-fluor-1,4-dihydro-4-oxo-1,8-naphthyridin-3-carbonsäure-ethylester, 1-Cyclopropyl-6,7,8-trifluor-1,4-dihydro-4-oxo-3-chinolincarbonsäureethylester, 9,10-Difluor-2,3-dihydro-3-methyl-7-oxo-7H-pyrido-[1,2,3-de][1,4]benzoxazin-6-carbonsäure, 8,9-Difluor-6,7-dihydro-5-methyl-1-oxo-1H,5H-benzo[i,j]-chinolizin-2-carbonsäure, 7-Chlor-6-fluor-1-phenyl-1,4-dihydro-4-oxo-1,8-naphthyridin-3-carbonsäure, 7-Chlor-6-fluor-1-(4-fluorphenyl)-1,4-dihydro-4-oxo-1,8-naphthyridin-3-carbonsäureethylester, 6,7,8-Trifluor-1,4-dihydro-1-methylamino-4-oxo-3-chinolincarbonsäure, 1-Amino-6,7,8-trifluor-1,4-dihydro-4-oxo-3-chinolincarbonsäure, 6,7,8-Trifluor-1,4-dihydro-1-dimethylamino-4-oxo-3-chinolincarbonsäure, 6,7-Difluor-1-(4-fluorphenyl)-1,4-dihydro-8-methyl-4-oxo-3-chinolincarbonsäure, 7-Chlor-6-fluor-1-(4-fluorphenyl)-1,4-dihydro-4-oxo-3-chinolincarbonsäure, 7-Chlor-6-fluor-1-(2,4-difluorphenyl)-1,4-dihydro-4-oxo-3-chinolincarbonsäure, 6,7,8-Trifluor-1-(4-fluorphenyl)-1,4-dihydro-4-oxo-3-chinolincarbonsäure, 1-Cyclopropyl-6,7-difluor-1,4-dihydro-5-methyl-4-oxo-3-chinolincarbonsäure, 7-Chlor-1-cyclopropyl-6-fluor-1,4-dihydro-5-methyl-4-oxo-1,8-naphthyridin-3-carbonsäure, 7-Chlor-6-fluor-1-(2,4-difluorphenyl)-1,4-dihydro-5-methyl-4-oxo-1,8-naphthyridin-3-carbonsäure, 6,7-Difluor-1,4-dihydro-1-(3-oxetanyl)-4-oxo-3-chinolincarbonsäure, 6,7,8-Trifluor-1,4-dihydro-1-(3-oxetanyl)-4-oxo-3-chinolincarbonsäure, 1-(Bicyclo[1.1.1]pent-1-yl)-6,7,8-trifluor-1,4-dihydro-4-oxo-3-chinolincarbonsäure, 7-Chlor-1-(1,1-dimethylpropargyl)-6-fluor-1,4-dihydro-4-oxo-1,8-naphthyridin-3-carbonsäure, 6,7,8-Trifluor-1-(2,4-difluorphenyl)-1,4-dihydro-4-oxo-3-chinolincarbonsäure, 6,7,8-Trifluor-1,4-dihydro-4-oxo-1-phenyl-3-chinolincarbonsäure, 7-Chlor-1-ethyl-6-fluor-1,4-dihydro-4-oxo-1,8-naphthyridin-3-carbonsäure, 6,7-Difluor-1,4-dihydro-4-oxo-1-vinyl-3-chinolincarbonsäure, 1-Cyclopropyl-5,6,7,8-tetrafluor-1,4-dihydro-4-oxo-3-chinolincarbonsäure, 5-Amino-1-cyclopropyl-6,7,8-trifluor-1,4-dihydro-4-oxo-3-chinolincarbonsäure, 1-Cyclopropyl-6,7,8-trifluor-1,4-dihydro-5-hydroxy-4-oxo-3-chinolincarbonsäure, 1-Cyclopropyl-6,7-difluor-1,4-dihydro-8-methoxy-4-oxo-3-chinolincarbonsäure, 7-Chlor-1-(2,4-difluorphenyl)-6-fluor-1,4-dihydro-4-oxo-1,8-naphthyridin-3-carbonsäureethylester, 1-Cyclopropyl-6,7-difluor-1,4-dihydro-4-oxo-8-vinyl-3-chinolincarbonsäure, 1-Cyclopropyl-8-ethinyl-6,7-difluor-1,4-dihydro-4-oxo-3-chinolincarbonsäure, 9,10-Difluor-3-methyl-7-oxo-2,3-dihydro-7H-pyrido[1,2,3-d,e][1,3,4]benzoxadiazin-6-carbonsäure, 8-Amino-9,10-difluor-3-methyl-7-oxo-2,3-dihydro-7H-pyrido[1,2,3-d,e][1,3,4]benzoxadiazin-6-carbonsäure, 7,8-Difluor-5-oxo-9,1-[(N-methylimino)methano]-5H-thiazolo[3,2-a]-chinolin-4-carbonsäure, 7,8-Difluor-5-oxo-9,1-[(N-ethylimino)methano]-5H-thiazolo[3,2-a]chinolin-4-carbonsäure, 7,8-Difluor-5-oxo-9,1-(epoxymethano)-5H-thiazolo[3,2-a]-chinolin-4-carbonsäure, 7,8-Difluor-5-oxo-9,1-(epithiomethano)-5H-thiazolo[3,2-a]-chinolin-4-carbonsäure, 7,8-Difluor-1-methyl-5-oxo-5H-thiazolo[3,2-a]-chinolin-4-carbonsäure, 8-Brom-6,7-difluor-1-(cis-2-fluorcyclopropyl)-1,4-dihydro-4-oxo-3-chinolincarbonsäure, 8-Chlor-6,7-difluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-4-oxo-3-chinolincarbonsäure, 6,7,8-Trifluor-1-(cis-2-fluorcyclopropyl)-1,4-dihydro-4-oxo-3-chinolincarbonsäure, 5,6,7,8-Tetrafluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-8-methyl-4-oxo-3-chinolincarbonsäure, 8-Ethinyl-6,7-difluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-4-oxo-8-trifluormethyl-3-chinolincarbonsäure, 6,7-Difluor-1-(cis-2-fluorcyclopropyl)-8-difluormethoxy-1,4-dihydro-4-oxo-3-chinolincarbonsäure, 6,7-Difluor-1-(cis-2-fluorcyclopropyl)-1,4-dihydro-8-methoxy-4-oxo-3-chinolincarbonsäure, 6,7-Difluor-1-(cis-2-fluorcyclopropyl)-1,4-dihydro-5-methyl-4-oxo-3-chinolincarbonsäure, 5-Amino-6,7,8-trifluor-1-(cis-2-fluorcyclopropyl)-1,4-dihydro-4-oxo-3-chinolincarbonsäure, 8-Brom-6,7-difluor-1-[(1R,2S)-2-fluorcyclopropyl]-1,4-dihydro-4-oxo-3-chinolincarbonsäure, 8-Chlor-6,7-difluor-1-[(1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-4-oxo-3-chinolincarbonsäure, 6,7,8-Trifluor-1-[(1R,2S)-2-fluorcyclopropyl]-1,4-dihydro-4-oxo-3-chinolincarbonsäure, 5,6,7,8-Tetrafluor-1-[(1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-8-methyl-4-oxo-3-chinolincarbonsäure,8-Ethinyl-6,7-difluor-1-[(1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-4-oxo-8-trifluormethyl-3-chinolincarbonsäure, 6,7-Difluor-1-[(1R,2S)-2-fluorcyclopropyl]-8-difluormethoxy-1,4-dihydro-4-oxo-3-chinolincarbonsäure, 6,7-Difluor-1-[(1R,2S)-2-fluorcyclopropyl]-1,4-dihydro-8-methoxy-4-oxo-3-chinolincarbonsäure, 6,7-Difluor-1-[(1R,2S)-2-fluorcyclopropyl]-1,4-dihydro-5-methyl-4-oxo-3-chinolincarbonsäure, 5-Amino-6,7,8-Trifluor-1-[(1R,2S)-2-fluorcyclopropyl]-1,4-dihydro-4-oxo-3-chinolincarbonsäure, 6,7-Difluor-4-oxo-4H-[1,3]thiazeto[3,2-a]chinolin-3-carbonsäure, 6,7-Difluor-1-methyl-4-oxo-4H-[1,3]thiazeto[3,2-a]chinolin-3-carbonsäure, 6,7-Difluor-1-flourmethyl-4-oxo-4H-[1,3]thiazeto[3,2-a]chinolin-3-carbonsäure, 1-Cyclopropyl-6,7-difluor-1,4-dihydro-8-methyl-4-oxo-3-chinolincarbonsäure B(O-CO-CH₃)₂-Chelat.

Die als Ausgangsverbindungen benötigten bicyclischen Amine der Formel (III) sind neu. Sie können nach den in Schema 1 aufgeführten Methoden hergestellt werden: Ausgehend von einem 2,5-Dihydropyrrolcarbonsäurealkylester (1) kann man mit geeigneten Dienen die Diels-Alder-Addukte (2) beziehungsweise (3) aufbauen. An Stelle von Dienen können auch geeignete Diensynthone, wie zum Beispiel α-Pyron verwendet werden. Aus (2) läßt sich durch Addition von Brom in inerten Lösungsmitteln und nachfolgende Dehydrobromierung mit starken Basen wie zum Beispiel Kalium-tert.-butylat, 1,5-Diazabicyclo[4.3.0]non-5-en, 1,8-Diazabicyclo[5.4.0]undec-7-en oder Ethyl-diisopropylamin das Dien (4) aufbauen, welches auch durch Säurebehandlung aus dem Zwischenprodukt (3) erhalten werden kann. Der Diencarbonsäurealkylester (4) wird zur Carbonsäure hydrolysiert, die beispielsweise durch Hoffmann- oder Curtius-Abbau über das Urethan (5) als Zwischenprodukt zum Amin (6) abgebaut werden kann. Ferner läßt sich das Urethan (5) selektiv am Urethanstickstoff zum Alkylurethan (7) alkylieren, in das nach selektiver Abspaltung der Urethangruppe gegebenenfalls eine zweite Alkylgruppe eingeführt und danach in (8) überführt werden kann. Durch Reduktion des Diencarbonsäureesters (4) mit komplexen Hydriden kann über die Zwischenstufe (9) die Hydroxymethylverbindung (12) aufgebaut werden. Ferner können aus (9) nach Aktivierung der Hydroxylgruppe beispielsweise durch Überführung in ein O-Tosylat oder ein O-Mesylat und nachfolgende nukleophile Substitutionsreaktion mit Aminen oder Aziden, die anschließend reduziert werden müssen, über Amine der Struktur (10) die Amine der Struktur (11) hergestellt werden. An Stelle des Carbonsäurealkylesters (1) kann auch ein analoges 2,5-Dihydropyrrol-3-carbonitril für die Synthese eingesetzt werden, aus dem beispielsweise durch eine ähnliche Reaktionssequenz (Diels-Alder-Reaktion, Reduktion) 3a-Aminomethyl-1,2,3,7a-tetrahydro-isoindol hergestellt werden kann.

Die Zwischenprodukte der Strukturen (6), (8), (11) und (12) entsprechen der allgemeinen Formel (III).

1-Amino-8-azabicyclo[4.3.0]nona-2,4-dien kann auch hergestellt werden, indem man 8-Azabicyclo[4.3.0]nona-2,4-dien-1-carboxamid-8-carbonsäuremethylester durch einen Hofmann-Abbau, beispielsweise mit Natriumhypochlorit, Natriumhypobromit oder Jodosobenzol zum 1-Amino-8-azabicyclo[4.3.0]nona-2,4-dien-8-carbonsäuremethylester umsetzt und anschließend die Carbamatschutzgruppe durch Behandlung mit Säuren oder Basen abspaltet. 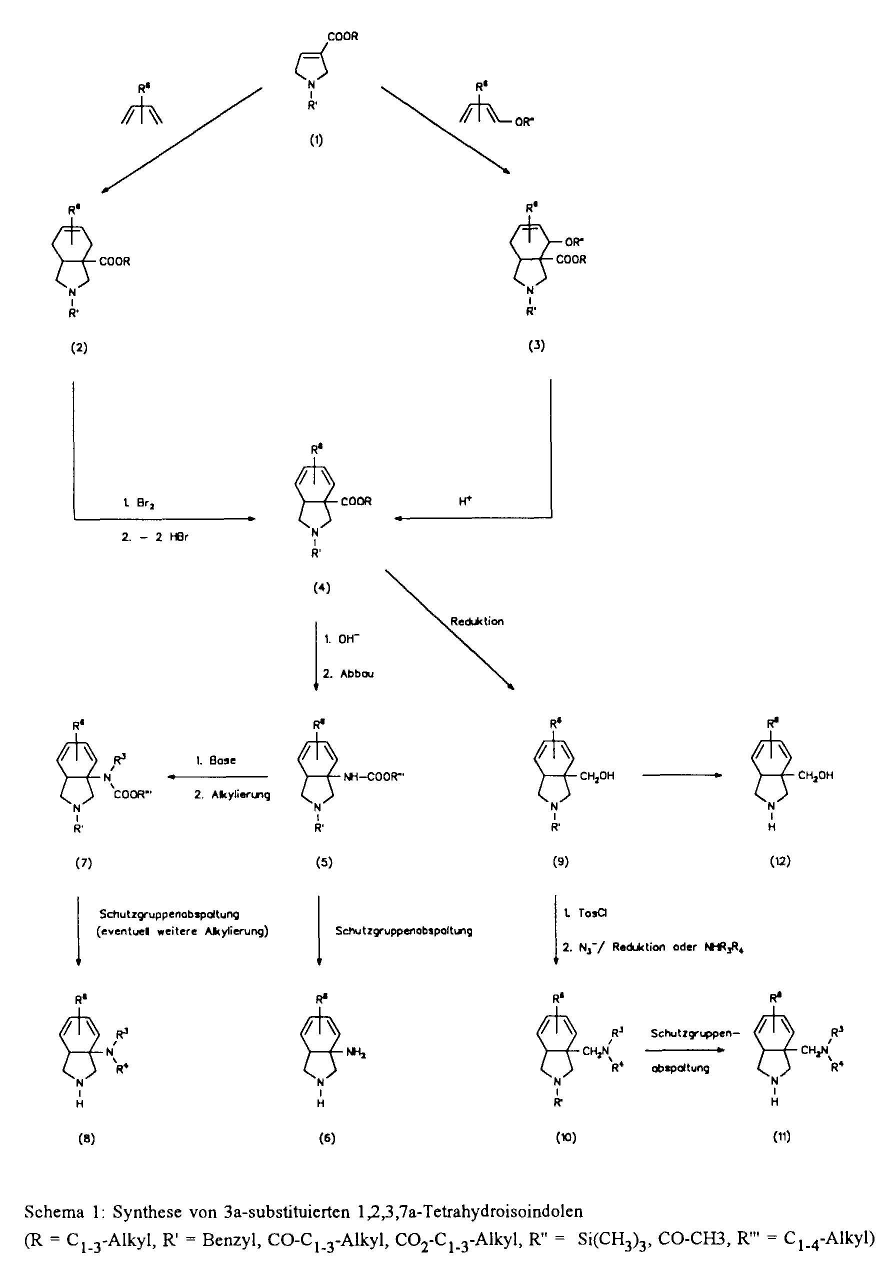 Als Beispiele für zweifach ungesättigte bicyclische Amine der Formel (III) seien genannt:
1,2,3,7a-Tetrahydro-isoindol-3a-ylamin, 4-Methyl-1,2,3,7a-tetrahydro-isoindol-3a-ylamin, 5-Methyl-1,2,3,7a-tetrahydro-isoindol-3a-ylamin, 6-Methyl-1,2,3,7a-tetrahydro-isoindol-3a-ylamin, 7-Methyl-1,2,3,7a-tetrahydro-isoindol-3a-ylamin, 3a-Methylamino-1,2,3,7a-tetrahydro-isoindol, 3a-Dimethylamino-1,2,3,7a-tetrahydro-isoindol, 3a-tert.-Butoxycarbonylamino-1,2,3,7a-tetrahydro-isoindol, 3a-Aminomethyl-1,2,3,7a-tetrahydro-isoindol, 3a-Methylaminomethyl-1,2,3,7a-tetrahydro-isoindol, 3a-Dimethylaminomethyl-1,2,3,7a-tetrahydro-isoindol, 3a-Hydroxy-1,2,3,7a-tetrahydro-isoindol,3a-Hydroxymethyl-1,2,3,7a-tetrahydro-isoindol.

Die enantiomerenreinen Ausgangsverbindungen der Formel (III) können nach folgenden Verfahren hergestellt werden:
1. Die racemischen bicyclischen Amine (III) können mit enantiomerenreinen Säuren, zum Beispiel Carbonsäuren oder Sulfonsäuren wie N-Acetyl-L-glutaminsäure, N-Benzoyl-L-alanin, 3-Brom-campher-9-sulfonsäure, Campher-3-carbonsäure, cis-Camphersäure, Campher-10-sulfonsäure, O,O'-Dibenzoylweinsäure, D- oder L-Weinsäure, Mandelsäure, α-Methoxy-phenylessigsäure, 1-Phenyl-ethansulfonsäure, α-Phenyl-bernsteinsäure zu einem Gemisch der diastereomeren Salze umgesetzt werden, die sich durch fraktionierte Kristallisation zu den diastereomerenreinen Salzen trennen lassen (siehe P. Newman, Optical Resolution Procedures for Chemical Compounds, Volume 1). Durch Behandlung dieser Salze mit Alkali- oder Erdalkalihydroxyden lassen sich die enantiomerenreinen Amine freisetzen.
2. In ähnlicher Weise wie unter 1. beschrieben läßt sich eine Racematspaltung der basischen Zwischenstufen (siehe *Schema 1*), die bei der Herstellung der racemischen bicyclischen Amine auftreten, mit den oben aufgeführten enantiomerenreinen Säuren durchführen.
3. Sowohl die racemischen Amine (III) als auch die in *Schema 1* aufgeführten Zwischenstufen können, gegebenenfalls nach Acylierung, über chirale Trägermaterialien chromatographisch getrennt werden (siehe zum Beispiel G. Blaschke, Angew. Chem. 92, 14 [1980]).
4. Die racemischen Amine (III) können auch durch chemische Verknüpfung mit chiralen Acylresten in Diastereomerengemische überführt werden, die sich durch Destillation, Kristallisation oder Chromatographie in die diastereomerenreinen Acylderivate trennen lassen, aus denen sich durch Verseifung die enantiomerenreinen Amine isolieren lassen. Beispiele für Reagentien zur Verknüpfung mit chiralen Acylresten sind: α-Methoxy-α-trifluormethyl-phenylacetylchlorid, Menthylisocyanat, D- oder L-α-Phenyl-ethyl-isocyanat, Chlorameisensäure-menthylester, Campher-10-sulfonsäurechlorid.
5. Im Verlauf der Synthese der bicyclischen Amine (III) können statt achiraler auch chirale Schutzgruppen eingeführt werden. Man gelangt auf diese Weise zu Diastereomerengemischen, die sich auftrennen lassen. Zum Beispiel kann bei der Synthese der Zwischenstufe (4) in *Schema 1* der Benzylrest durch den R-oder S-konfigurierten α-Phenylethylrest beziehungsweise die Alkoholkomponente des Esters (4) durch einen enantiomerenreinen Alkohol, wie zum Beispiel Menthol oder Pantolacton ersetzt werden.

Die Umsetzung von (II) mit (III), bei der die Verbindungen (III) auch in Form ihrer Salze, wie zum Beispiel der Hydrochloride, eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol oder Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 160°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 5 Mol der Verbindung (III) ein.

Freie Aminogruppen können während der Umsetzung durch eine geeignete Aminoschutzgruppe, wie zum Beispiel durch den tert.-Butoxycarbonylrest oder eine Azomethin-Schutzgruppe, geschützt und nach Beendigung der Reaktion wieder freigesetzt werden.

Die erfindungsgemäßen Verbindungen der Formel (I), in welcher R² für CH₂NO₂ oder Dialkoxycarbonylmethyl steht, können auch erhalten werden, indem man eine Verbindung der Formel (I), in welcher R² für OH steht, mit einem Aktivierungsmittel wie Carbonyldiimidazol in einem Lösungsmittel wie Tetrahydrofuran, Dichlormethan oder Chloroform umsetzt und anschließend mit einer CH-aciden Verbindung wie Nitromethan oder Malonsäuredialkylester umsetzt. Diese Umsetzung wird bevorzugt in einem Lösungsmittel wie Tetrahydrofuran in Gegenwart einer Base (Natriumhydrid, Kaliumcarbonat, Natriumcarbonat) durchgeführt.

Die Herstellung von erfindungsgemäßen Verbindungen der Formel (I), in welcher X² = NH₂ bedeutet, gelingt auch durch Umsetzung von Verbindungen der Formel (I), in welcher X² = F bedeutet, mit Ammoniak in polaren Lösungsmitteln wie Dimethylsulfoxid bei Temperaturen von 50°C bis 120°C bei Normaldruck oder durch Erhitzen im Autoklaven. Die Herstellung von erfindungsgemäßen Verbindungen der Formel (I), in welcher A = C-OCH₃ bedeutet, gelingt auch durch Umsetzung von Verbindungen der Formel (I), in welcher A = C-F bedeutet, mit Alkalimethylaten, wie zum Beispiel Natriummethylat, in Lösungsmitteln wie zum Beispiel Dimethylformamid, Glykoldimethylether, Dioxan, Tetrahydrofuran, Dimethylsulfoxid, Hexamethylphosphorsäuretrisamid oder Alkoholen bei Temperaturen von 20°C bis 150°C. Die Umsetzung kann bei Verwendung tiefsiedender Lösungsmittel auch im Autoklaven unter Druck durchgeführt werden. Durch Zugabe von Kronenethern, wie zum Beispiel 15-crown-5 oder 18-crown-6 läßt sich die Reaktion beschleunigen.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20 bis 180°C, vorzugsweise etwa 60 bis 120°C, umgesetzt. Das entstehende Reaktionswasser kann auch durch azeotrope Destillation mit Chloroform, Tetrachlormethan oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten Ester, wie zum Beispiel (5-Methyl-2-oxo-1,3-dioxol-4-yl-methyl)-ester, werden durch Umsetzung eines Alkalisalzes der zugrundeliegenden Carbonsäure, die gegebenenfalls am N-Atom durch eine Schutzgruppe geschützt sein kann, mit 4-Brommethyl- oder 4-Chlormethyl-5-methyl-1,3-dioxol-2-on in einem Lösungsmittel wie Dimethylformamid, Dimethylacetamid, N-Methyl-pyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen in überschüssiger wäßriger Säure und Ausfällen des Salzes mit einem mit Wasser mischbaren Lösungsmittel wie Methanol, Ethanol, Aceton oder Acetonitril. Man kann auch äquivalente Menge Betain und Säure in Wasser lösen und die Lösung lyophilisieren oder in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, 2-Hydroxyglutarsäure, Methansulfonsäure, 4-Toluolsulfonsäure, Galacturonsäure, Glucuronsäure, 5-Oxotetrahydrofuran-2-carbonsäure, Embonsäure, Glutaminsäure oder Asparaginsäure zu verstehen.

Die Alkali- und Erdalkalisalze der erfindungsgemäßen Carbonsäuren werden beispielsweise durch Lösen des Betains in überschüssiger Alkali- oder Erdalkalilauge, Filtration vom ungelösten Betain und Eindampfen bis zur Trockne erhalten. Pharmazeutisch geeignet sind Natrium-, Kalium- und Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die nachfolgend aufgeführten sowie die in den folgenden Tabellen aufgeführten Wirkstoffe hergestellt werden, die sowohl als Racemate oder als enantiomerenreine Verbindungen oder gegebenenfalls auch als Diastereomerengemische oder als diastereomerenreine Verbindungen vorliegen können:
8-(3a-Amino-1,2,3,7a-tetrahydro-isoindol-2-yl)-7-fluor-5-oxo-9,1-(epoxymethano)-5H-thiazolo[3,2-a]chinolin-4-carbonsäure, 7-Fluor-8-(3a-methylamino-1,2,3,7a-tetrahydro-isoindol-2-yl)-5-oxo-9,1-(epoxymethano)-5H-thiazolo[3,2-a]chinolin-4-carbonsäure, 8-(3a-Aminomethyl-1,2,3,7a-tetrahydro-isoindol-2-yl)-7-fluor-5-oxo-9,1-(epoxymethano)-5H-thiazolo[3,2-a]chinolin-4-carbonsäure, 7-Fluor-8-(3a-methylaminomethyl-1,2,3,7a-tetrahydro-isoindol-2-yl)-5-oxo-9,1-(epoxymethano)-5H-thiazolo[3,2-a]chinolin-4-carbonsäure, 8-(3a-Amino-1,2,3,7a-tetrahydro-isoindol-2-yl)-7-fluor-5-oxo-9,1-[(N-methyl-imino)methano]-5H-thiazolo[3,2-a]chinolin-4-carbonsäure, 7-Fluor-8-(3a-methylamino-1,2,3,7a-tetrahydro-isoindol-2-yl)-5-oxo-9,1-[(N-methyl-imino)methano]-5H-thiazolo[3,2-a]chinolin-4-carbonsäure, 8-(3a-Aminomethyl-1,2,3,7a-tetrahydro-isoindol-2-yl)-7-fluor-5-oxo-9,1-[(N-methylimino)methano]-5H-thiazolo[3,2-a]chinolin-4-carbonsäure, 7-Fluor-8-(3a-methyl-aminomethyl-1,2,3,7a-tetrahydro-isoindol-2-yl)-5-oxo-9,1-[(N-methyl-imino)methano]-5H-thiazolo[3,2-a]chinolin-4-carbonsäure, 10-(3a-Amino-1,2,3,7a-tetrahydro-isoindol-2-yl)-9-fluor-3-methyl-7-oxo-2,3-dihydro-7H-pyrido[1,2,3-d,e]-[1,3,4]benzoxadiazin-6-carbonsäure, 9-Fluor-3-methyl-10-(3a-methylamino-1,2,3,7a-tetrahydro-isoindol-2-yl)-7-oxo-2,3-dihydro-7H-pyrido[1,2,3-d,e]-[1,3,4]benzoxadiazin-6-carbonsäure, 10-(3a-Aminomethyl-1,2,3,7a-tetrahydro-isoindol-2-yl)-9-fluor-3-methyl-7-oxo-2,3-dihydro-7H-pyrido[1,2,3-d,e]-[1,3,4]benzoxadiazin-6-carbonsäure, 8-Amino-10-(3a-amino-1,2,3,7a-tetrahydro-isoindol-2-yl)-9-fluor-3-methyl-7-oxo-2,3-dihydro-7H-pyrido[1,2,3-d,e][1,3,4]-benzoxadiazin-6-carbonsäure, 10-(3a-Dimethylaminomethyl-1,2,3,7a-tetrahydro-isoindol-2-yl)-9-fluor-3-methyl-7-oxo-2,3-dihydro-7H-pyrido[1,2,3-d,e][1,3,4]-benzoxadiazin-6-carbonsäure, 9-Fluor-3-methyl-10-(3a-methylamino-1,2,3,7a-tetrahydro-isoindol-2-yl)-7-oxo-2,3-dihydro-7H-pyrido[1,2,3-d,e][1,4]benzoxazin-6-carbonsäure, 10-(3a-Aminomethyl-1,2,3,7a-tetrahydro-isoindol-2-yl)-9-fluor-3-methyl-7-oxo-2,3-dihydro-7H-pyrido[1,2,3-d,e][1,4]benzoxazin-6-carbonsäure, 8-Amino-10-(3a-amino-1,2,3,7a-tetrahydro-isoindol-2-yl)-9-fluor-3-methyl-7-oxo-2,3- dihydro-7H-pyrido[1,2,3-d,e][1,4]benzoxazin-6-carbonsäure, 10-(3a-Amino-5-methyl-1,2,3,7a-tetrahydro-isoindol-2-yl)-9-fluor-3-methyl-7-oxo-2,3-dihydro-7H-pyrido[1,2,3-d,e][1,4]benzoxazin-6-carbonsäure, 7-(3a-Amino-1,2,3,7a-tetrahydro-isoindol-2-yl)-6-fluor-4-oxo-4H-[1,3]thiazeto[3,2-a]chinolin-3-carbonsäure, 7-(3a-Amino-1,2,3,7a-tetrahydro-isoindol-2-yl)-6-fluor-1-methyl-4-oxo-4H-[1,3]thiazeto-[3,2-a]chinolin-3-carbonsäure, 7-(3a-Amino-1,2,3,7a-tetrahydro-isoindol-2-yl)-6-fluor-1-fluormethyl-4-oxo-4H-[1,3]thiazeto[3,2-a]chinolin-3-carbonsäure, 7-(3a-Amino-1,2,3,7a-tetrahydro-isoindol-2-yl)-1-cyclopropyl-6-fluor-3-nitroacetyl-4-oxo-1,4-dihydrochinolin, 7-(3a-Amino-1,2,3,7a-tetrahydro-isoindol-2-yl)-1-cyclopropyl-6-fluor-8-methoxy-3-nitroacetyl-4-oxo-1,4-dihydrochinolin, 7-(3a-Amino-1,2,3,7a-tetrahydro-isoindol-2-yl)-1-cyclopropyl-6,8-difluor-3-nitroacetyl-4-oxo-1,4-dihydrochinolin, 7-(3a-Amino-1,2,3,7a-tetrahydro-isoindol-2-yl)-1-cyclopropyl-8-chlor-6-fluor-3-nitroacetyl-4-oxo-1,4-dihydrochinolin, 7-(3a-Amino-1,2,3,7a-tetrahydro-isoindol-2-yl)-1-cyclopropyl-3-(diethoxycarbonyl)acetyl-6-fluor-4-oxo-1,4-dihydrochinolin, 7-(3a-Amino-1,2,3,7a-tetrahydro-isoindol-2-yl)-1-cyclopropyl-3-(diethoxycarbonyl)acetyl-6-fluor-8-methoxy-4-oxo-1,4-dihydrochinolin, 7-(3a-Amino-1,2,3,7a-tetrahydro-isoindol-2-yl)-1-cyclopropyl-3-(diethoxycarbonyl)acetyl-6,8-difluor-4-oxo-1,4-dihydrochinolin, 7-(3a-Amino-1,2,3,7a-tetrahydro-isoindol-2-yl)-8-chlor-1-cyclopropyl-3-(diethoxycarbonyl)acetyl-6-fluor-4-oxo-1,4-dihydrochinolin.

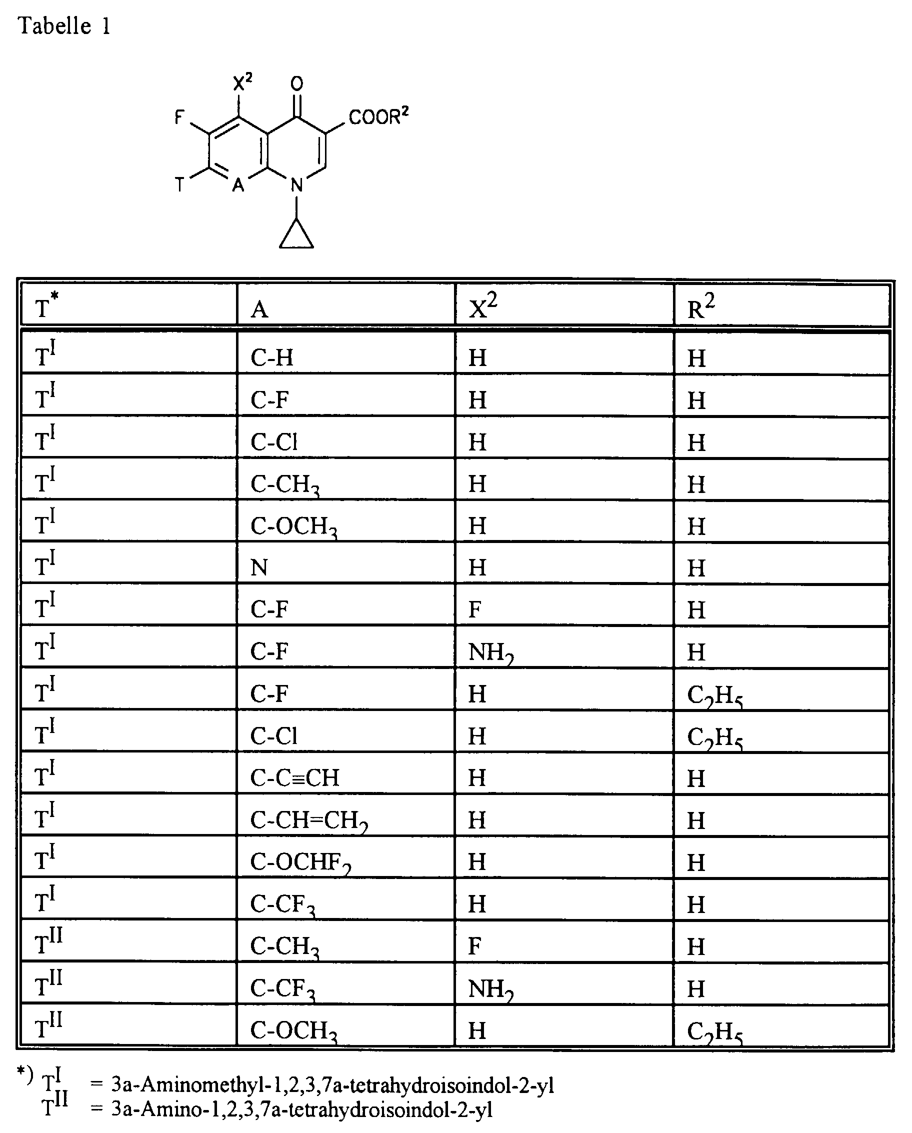

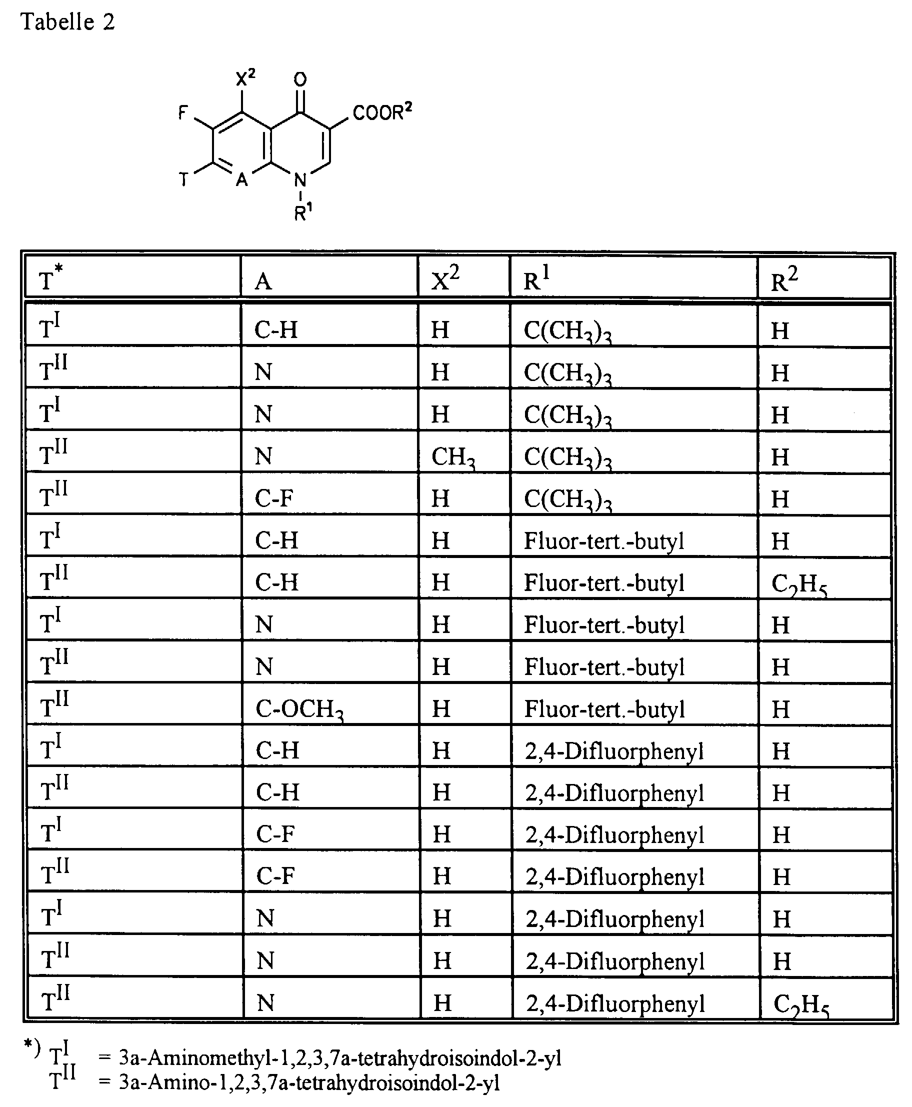

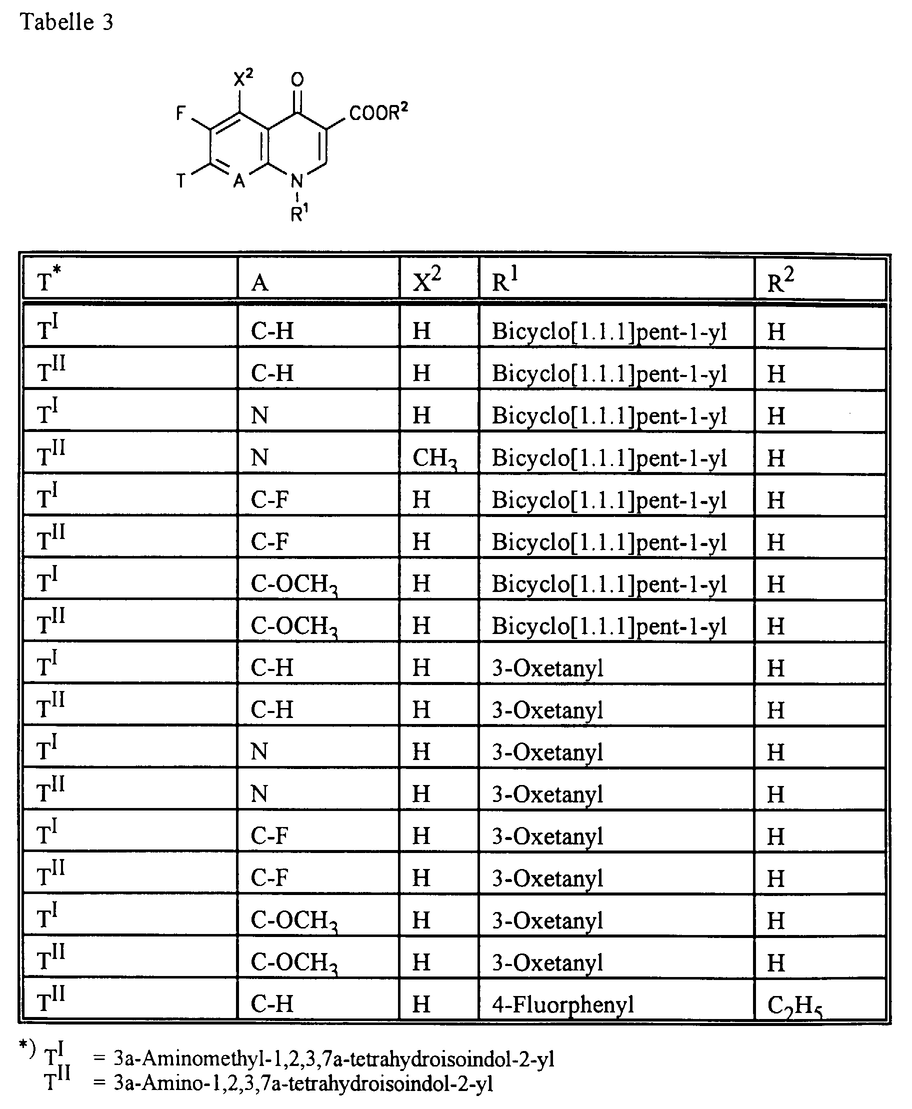

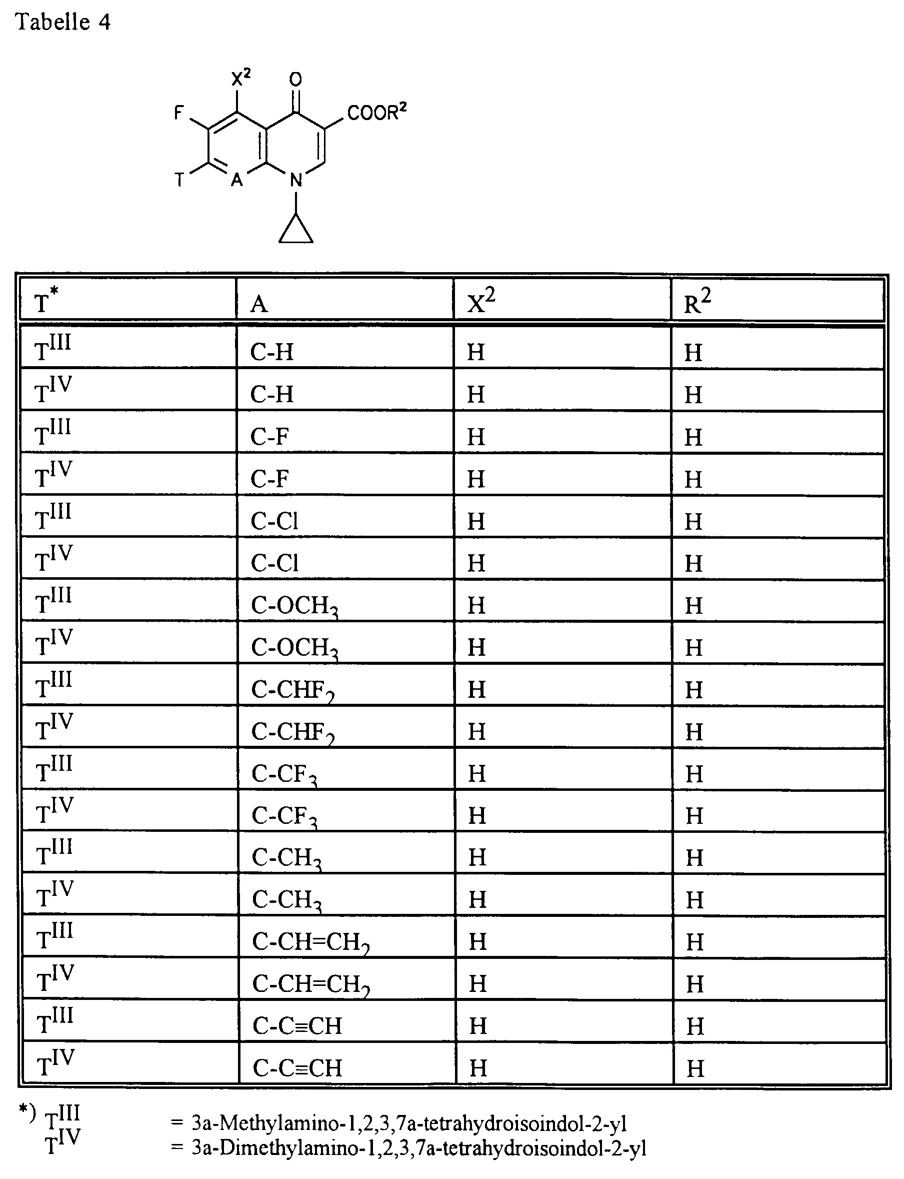

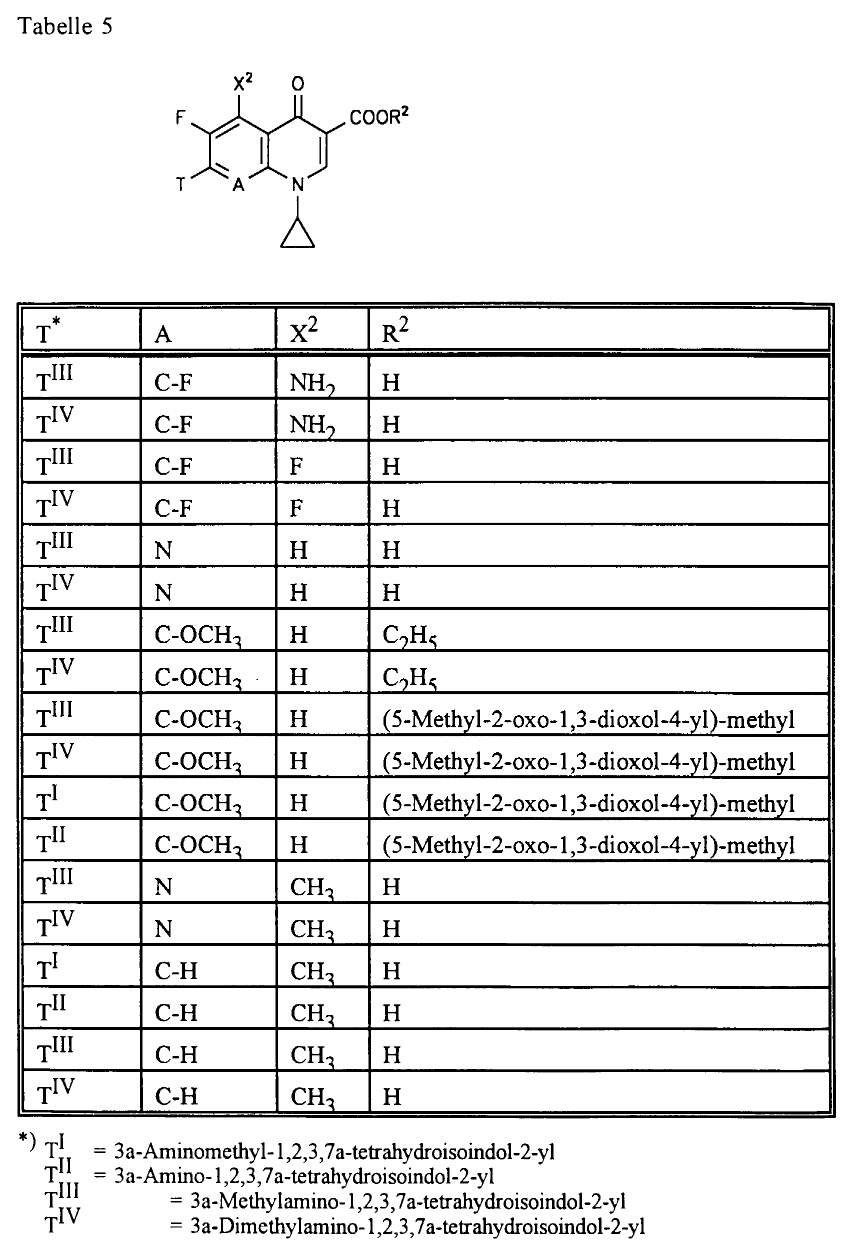

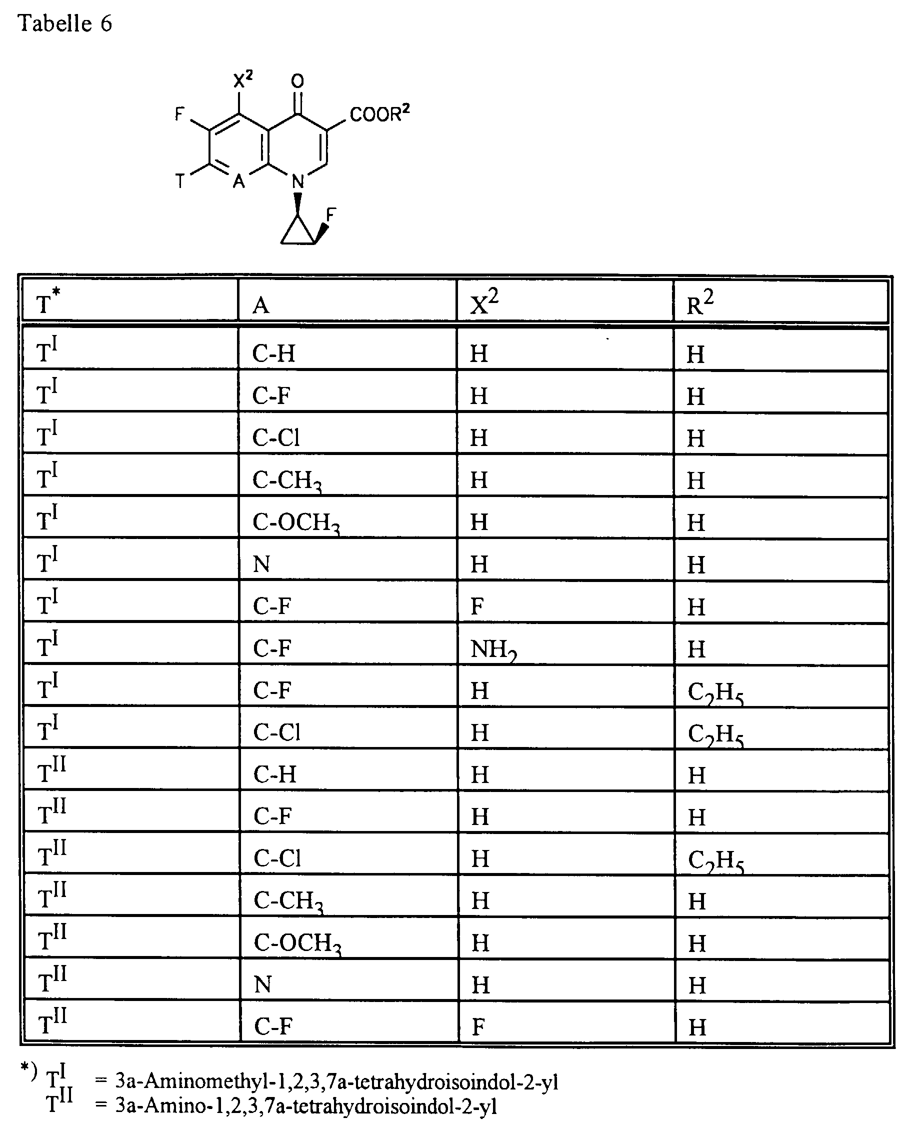

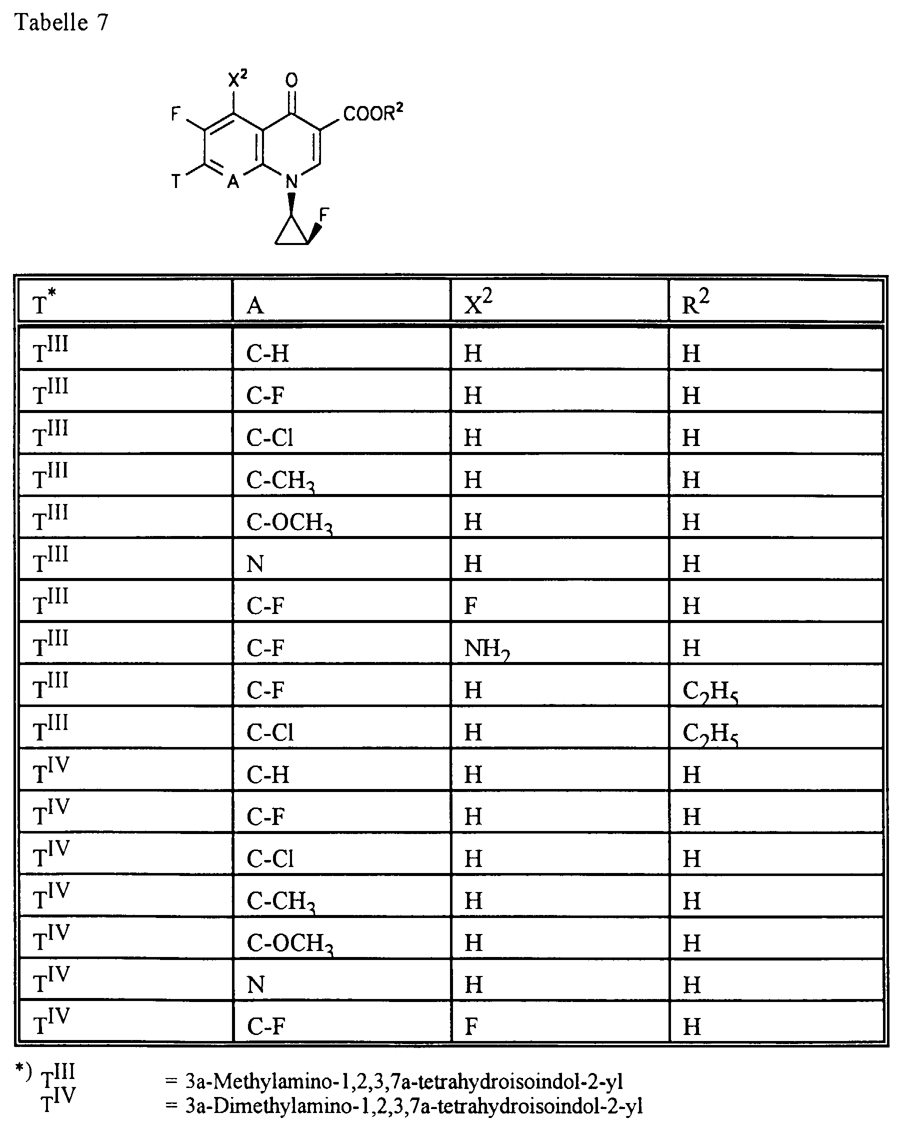

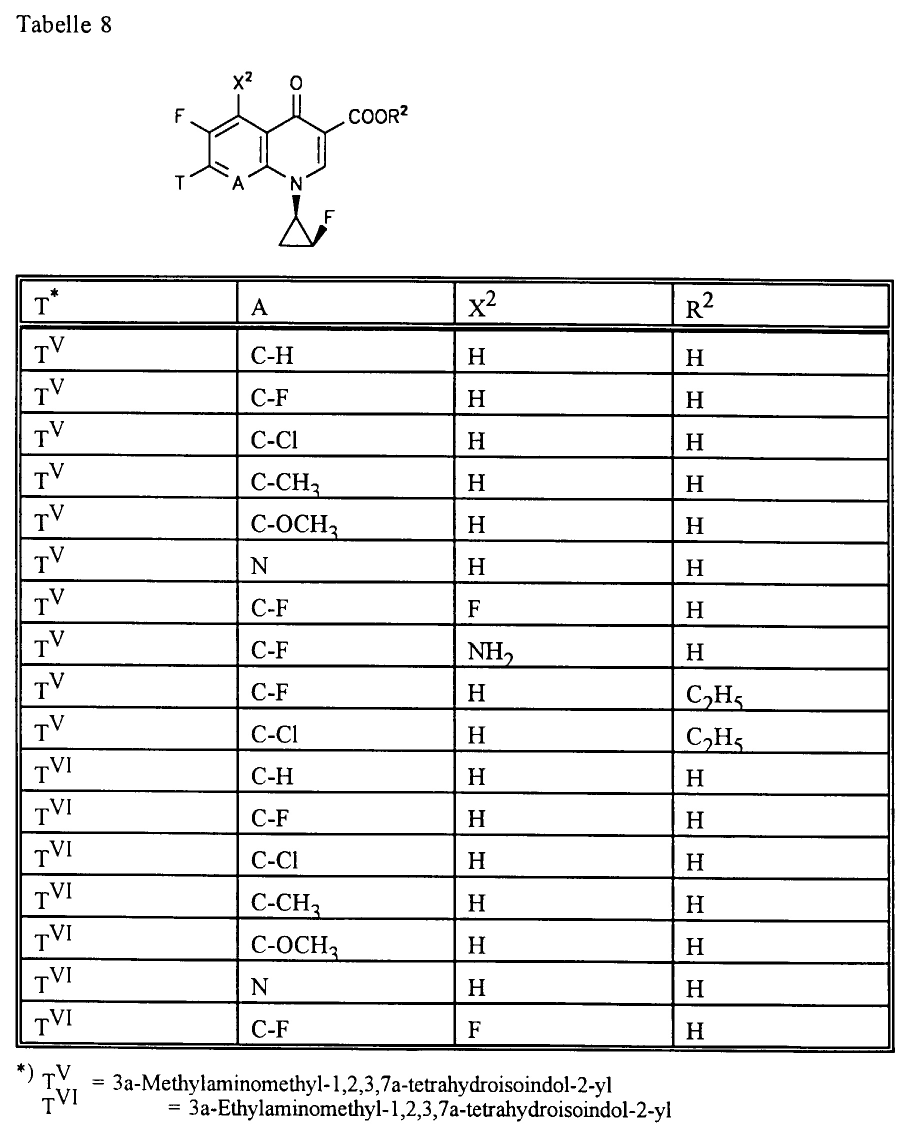

Die erfindungsgemäßen Verbindungen wirken stark antibiotisch und zeigen bei geringer Toxizität ein breites antibakterielles Spektrum gegenüber grampositiven und gramnegativen Keimen, vor allem auch gegenüber solchen, die gegen verschiedene Antibiotika, wie zum Beispiel Penicilline, Cephalosporine, Aminoglykoside, Sulfonamide, Tetracycline sowie gegen handelsübliche Chinolone, resistent sind. Die erfindungsgemäßen Verbindungen zeichnen sich insbesondere dadurch aus, daß sie im Vergleich zu den Verbindungen nach dem Stand der Technik geringere Interaktionen mit Säugetier-DNA aufweisen.

Diese wertvollen Eigenschaften ermöglichen ihre Verwendung als chemotherapeutische Wirkstoffe in der Medizin sowie in der Veterinärmedizin. Ferner können sie als Stoffe zur Konservierung von anorganischen und organischen Materialien, zum Beispiel von Polymeren, Schmiermitteln, Farben, Fasern, Leder, Papier und Holz, von Lebensmitteln und von Wasser verwendet werden.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch eine verstärkte Wirkung auf ruhende Keime aus. Bei ruhenden Bakterien, also Bakterien, die kein nachweisbares Wachstum zeigen, wirken die Verbindungen stark bakterizid. Dies bezieht sich nicht nur auf die einzusetzende Menge, sondern auch auf die Geschwindigkeit der Abtötung. Solche Ergebnisse konnten bei grampositiven und gramnegativen Bakterien, insbesondere bei Staphylococcus aureus, Pseudomonas aeruginosa, Enterococcus faecalis und Escherichia coli beobachtet werden.

Besonders wirksam sind die erfindungsgemäßen Verbindungen gegen typische und atypische Mykobakterien und Helicobacter pylori sowie gegen bakterienähnliche Mikroorganismen, wie zum Beispiel Mykoplasmen und Rickettsien. Sie sind daher besonders gut in der Human- und Tiermedizin zur Prophylaxe und Chemotherapie von lokalen und systemischen Infektionen geeignet, die durch diese Erreger hervorgerufen werden.

Die Verbindungen eignen sich ferner besonders gut zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die erfindungsgemäßen Verbindungen können auch mit β-Lactamderivaten wie zum Beispiel Cephalosporinen oder Penemen über kovalente Bindungen zu sogenannten Dual-Action-Derivaten verknüpft werden.

In den folgenden Tabellen 9 und 10 sind die minimalen Hemmkonzentrationen als Maß für die antibakterielle Wirksamkeit und die ID₅₀-Werte als Maß für die Interaktionen mit Säugetier-DNA einer Substanz sowohl für erfindungsgemäße Verbindungen als auch für Referenzverbindungen aus dem Stand der Technik (EP 520 240) aufgeführt. Diese Daten belegen, daß die erfindungsgemäßen Verbindungen bei hoher antibakterieller Wirksamkeit wesentlich geringere Interaktionen mit Säugetier-DNA aufweisen.

Die minimalen Hemmkonzentrationen (MHK) wurden durch ein Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt circa 10⁴ koloniebildende Partikel enthielt. Die beimpften Agarplatten wurden bei 37°C bebrütet und das Keimwachstum nach circa 20 Stunden abgelesen. Der MHK-Wert (µg/ml) gibt die niedrigste Wirkstoffkonzentration an, bei der mit bloßem Auge kein Wachtum zu erkennen war.

Unter ID₅₀ versteht man die Konzentration eines Stoffes, bei der die DNA-Synthese in Zellen aus Ovarien des chinesischen Hamsters (CHO-KI) um 50 % gehemmt wird. Dieser Wert wird nach Inkubation der entsprechenden Substanzen in abfallenden Verdünnungsstufen über definierte Zeiträume bestimmt. Dazu wird mittels fluorophotometrischer Methoden die DNA-Synthese in CHO-KI-Zellen im Vergleich zu Kontrollen ermittelt.

**Tabelle 9**

| MHK-Werte (µg/ml) und ID₅₀-Werte von erfindungsgemäßen Wirkstoffen | | | | | | |
|---|---|---|---|---|---|---|
| | | Beispiel | | | | |
| Species | Strain | 2 | 3 | 4 | 8 | 9 |
| E. coli | Neumann | ≤0,015 | ≤0,015 | ≤0,015 | ≤0,015 | ≤0,015 |
| Staph. aureus | 133 | 0,06 | ≤0,015 | ≤0,015 | 0,03 | 0,06 |
| Staph. aureus | ICB 25701 | 2 | 0,25 | 0,5 | 0,5 | 8 |
| Ps. aeruginosa | Walter | 1 | 0,5 | 0,5 | 0,5 | 1 |
| Bac. fragilis | ES 25 | 1 | 0,125 | 2 | 0,5 | 4 |
| ID₅₀ (µg/ml) | | 32 | 32 | >64 | 16 | 32 |

**Tabelle 10**

| MHK-Werte (µg/ml) und ID₅₀-Werte von Wirkstoffen aus dem Stand der Technik | | | | |
|---|---|---|---|---|
| | | Beispiele aus EP 520 240 | | |
| Species | Strain | 35 Ref. 1 | Ref. 2 | 36 Ref. 3 |
| E. coli | Neumann | 0,015 | 0,015 | 0,015 |
| Staph. aureus | 133 | 0,015 | 0,015 | 0,015 |
| Staph. aureus | ICB 25701 | 0,06 | 0,015 | 0,015 |
| Ps. aeruginosa | Walter | 0,5 | 1 | 0,5 |
| Bac. fragilis | ES 25 | 0,5 | 0,25 | 0,125 |
| ID₅₀ (µg/ml) | | 0,015 | 0,1 | 0,1 |
| Ref.1: 7-(4-Amino-7-methyl-1,3,3a,4,7,7a-hexahydro-isoindol-2-yl)-1-cyclopropyl-6,8-difluor-1,4-dihydro-4-oxo-3-chinolincarbonsäure, Ref.2: 7-(4-Amino-7-methyl-1,3,3a,4,7,7a-hexahydro-isoindol-2-yl)-1-cyclopropyl-6-fluor-1,4-dihydro-8-methoxy-4-oxo-3-chinolincarbonsäure, Ref.3: 7-(4-Amino-7-methyl-1,3,3a,4,7,7a-hexahydro-isoindol-2-yl)-8-chlor-1-cyclopropyl-6-fluor-1,4-dihydro-4-oxo-3-chinolincarbonsäure. | | | | |

### Herstellung der Zwischenprodukte

### Beispiel Z 1

A. 8-Benzyl-8-azabicyclo[4.3.0]non-3-en-1-carbonsäureethylester (2-Benzyl-1,2,3,4,7,7a-hexahydro-isoindol-3a-carbonsäureethylester)
   Man löst 231 g (1 mol) 1-Benzyl-2,5-dihydropyrrol-3-carbonsäureethylester und 10 g 4-tert.-Butylbrenzkatechin in 1500 ml Toluol, preßt im Autoklaven 20 bar Stickstoff auf und drückt dann 350 g 1,3-Butadien in den Autoklaven. Man erhitzt drei Tage auf 120°C, kühlt ab und entspannt, engt die Lösung ein und destilliert.
   Ausbeute: 264,9 g (87,6 % der Theorie),
   Siedepunkt: 127-140°C/0,1 mbar,
   Das Produkt ist nach gaschromatographischer Bestimmung 94 %ig.
B. 8-Azabicyclo[4.3.0]non-3-en-1,8-dicarbonsäure-1-ethylester-8-methylester (1,2,3,4,7,7a-Hexahydro-isoindol-2,3a-dicarbonsäure-3a-ethylester-2-methyl-ester)
   16,4 g (57,5 mmol) 94 %iger 8-Benzyl-8-azabicyclo[4.3.0]non-3-en-1-carbonsäureethylester werden in 130 ml absolutem Chloroform gelöst, 7,5 g Na₂CO₃ zugesetzt und dann 12 g (0,12 mol) Chlorameisensäuremethylester zugetropft. Man erhitzt über Nacht unter Rückfluß, saugt die Salze ab, engt das Filtrat ein und destilliert den Rückstand.
   Ausbeute: 14,4 g (90 % der Theorie),
   Siedepunkt: 122-126°C/0,2 mbar,
   Das Produkt ist nach gaschromatographischer Bestimmung 91 %ig.
C. 8-Azabicyclo[4.3.0]nona-2,4-dien-1,8-dicarbonsäure-1-ethylester-8-methylester (1,2,3,7a-Tetrahydro-isoindol-2,3a-dicarbonsäure-3a-ethylester-2-methylester)
   Zu 46 g (0,17 mol) 94 %igem 8-Azabicyclo[4.3.0]non-3-en-1,8-dicarbonsäure-1-ethylester-8-methylester in 200 ml absolutem Chloroform tropft man unter Kühlung mit einem Wasserbad 30 g (0,187 mol) Brom und rührt zwei Stunden bei Raumtemperatur. Man engt ein, nimmt in 1 l absolutem Toluol auf und setzt 61 g (0,4 mol) 1,8-Diazabicyclo[5.4.0]undec-7-en hinzu. Man erhitzt drei Stunden unter Rückfluß, dekantiert nach dem Abkühlen von den ausgefallenen Kristallen, wäscht die Lösung mit Wasser, trocknet über MgSO₄, engt ein und destilliert.
   Ausbeute: 22,3 g (50 % der Theorie),
   Siedepunkt: 125-135°C/0,15 mbar,
   Das Produkt ist gaschromatographisch 95,5 %ig.
D. 8-Azabicyclo[4.3.0]nona-2,4-dien-1,8-dicarbonsäure-8-methylester (1,2,3,7a-Tetrahydro-isoindol-2,3a-dicarbonsäure-2-methylester)
   1. 22 g (83,6 mmol) 95,5 %iger 8-Azabicyclo[4.3.0]nona-2,4-dien-1,8-dicarbonsäure-1-ethylester-8-methylester werden mit 3,7 g (92,5 mmol) NaOH in 60 ml Methanol über Nacht unter Rückfluß erhitzt. Man engt die Lösung ein, nimmt in 40 ml Wasser auf und extrahiert einmal mit tert.-Butylmethylether. Die wäßrige Lösung wird mit 8 ml konzentrierter Salzsäure sauer gestellt und mehrfach mit Methylenchlorid extrahiert. Nach Trocknen über MgSO₄ wird eingeengt.
      Ausbeute: 20,9 g als Öl.
   2. Zu 170 g (0,61 mol, gaschromatographisch 90%ig) 8-Azabicyclo[4.3.0]nona-2,4-dien-1,8-dicarbonsäure-1-ethylester-8-methylester in 300 ml Tetrahydrofuran tropft man bei Raumtemperatur 32 g (0,76 mol) LiOH·H₂O in 300 ml Wasser und rührt über Nacht bei Raumtemperatur. Man destilliert das Tetrahydrofuran ab, extrahiert die wäßrige Lösung einmal mit tert.-Butylmethylether, stellt dann mit konzentrierter Salzsäure sauer und extrahiert mehrfach mit CH₂Cl₂. Die organischen Lösungen werden über MgSO₄ getrocknet, eingeengt und das kristallisierende Produkt aus Toluol umkristallisiert.
      Ausbeute: 115 g (84,5 % der Theorie),
      Schmelzpunkt: 107-110°C.
E. 1-Methoxycarbonylamino-8-azabicyclo[4.3.0]nona-2,4-dien-8-carbonsäuremethylester (3a-Methoxycarbonylamino-1,2,3,7a-tetrahydro-isoindol-2-carbonsäure-methylester)
   20,9 g roher 8-Azabicyclo[4.3.0]nona-2,4-dien-1,8-dicarbonsäure-8-methylester werden mit 9,6 g (92 mmol) Triethylamin, 26 g (107 mmol) Diphenylphosphorylazid und 5 g Methanol in 300 ml absolutem Toluol über Nacht unter Rückfluß erhitzt. Die Lösung wird mit Wasser gewaschen, über MgSO₄ getrocknet und eingeengt. Das Produkt wird roh weiter verarbeitet.
   Ausbeute: 20 g.
F. 1,2,3,7a-Tetrahydro-isoindol-3a-ylamin (1-Amino-8-azabicyclo[4.3.0]nona-2,4-dien)
   Man erhitzt 20 g rohen 1-Methoxycarbonylamino-8-azabicyclo[4.3.0]nona-2,4-dien-8-carbonsäuremethylester mit 75 g (0,235 mol) Ba(OH)₂·8 H₂O in 250 ml Wasser über Nacht unter Rückfluß. Man saugt das BaCO₃ ab, engt das Filtrat ein und kocht die Salzrückstände dreimal mit 1,4-Dioxan aus. Die Dioxanlösungen werden eingeengt und der Rückstand destilliert.
   Ausbeute: 5 g (43,9 % der Theorie bezogen auf Stufe D),
   Siedepunkt: 65°C/0,2 mbar.
G. (1S,6S)-8-Azabicyclo[4.3.0]nona-2,4-dien-1,8-dicarbonsäure-8-methylester ((3aS,7aS)-1,2,3,7a-Tetrahydro-isoindol-2,3a-dicarbonsäure-2-methylester)
   Enantiomerentrennung, Methode 1: 100 g (0,448 mol) 8-Azabicyclo[4.3.0]-nona-2,4-dien-1,8-dicarbonsäure-8-methylester löst man in einem Gemisch von 750 ml Diisopropylether und 750 ml Tetrahydrofuran und setzt 27 g (0,223 mol) R-(+)-1-Phenylethylamin hinzu. Man rührt über Nacht bei Raumtemperatur, saugt die Kristalle ab, wäscht sie mit kaltem Tetrahydrofuran und trocknet sie an der Luft.
   Ausbeute: 57 g Salz,
   [α]_{D}= + 156° (c = 1,2, Methanol),
   Die Kristalle werden aus 600 ml Isopropanol umkristallisiert.
   Ausbeute: 41 g (53,4 % der Theorie),
   [α]_{D} = + 197° (c = 1,1, Methanol).
   Enantiomerentrennung, Methode 2: 199 g (0,892 mol) 8-Azabicyclo[4.3.0]-nona-2,4-dien-1,8-dicarbonsäure-8-methylester werden in einer Mischung aus 800 ml Diisopropylether und 600 ml Tetrahydrofuran gelöst und 54 g (0,446 mol) S-(-)-1-Phenylethylamin hinzugefügt. Die Mischung wird über Nacht bei Raumtemperatur gerührt. Die ausgefallenen Kristalle werden abgesaugt und das isolierte Salz aus 1 l Isopropanol umkristallisiert.
   Ausbeute: 65,5 g (42,6 % der Theorie),
   [α]_{D}= -205,4° (c = 0,97, Methanol).
   Die vereinigten Mutterlaugen werden eingeengt und der Rückstand in 1 l tert.-Butyl-methyl-ether aufgenommen. Die Lösung wird mit einer Mischung aus 30 g konzentrierter Schwefelsäure und 200 ml Eiswasser extrahiert und die wäßrige Phase mit tert.-Butyl-methyl-ether reextrahiert. Die vereinigten tert.-Butyl-methyl-ether-Lösungen werden über MgSO₄ getrocknet und eingeengt.
   Ausbeute: 170,4 g.
   Dieser angereicherte (+)-8-Azabicyclo[4.3.0]nona-2,4-dien-1,8-dicarbonsäure-8-methylester wird in einer Mischung aus 800 ml Diisopropylether und 600 ml Tetrahydrofuran gelöst und 55 g R-(+)-1-Phenylethylamin zugesetzt. Das Salz wird abgesaugt, mit Tetrahydrofuran/Diisopropylether gewaschen und an der Luft getrocknet.
   Ausbeute: 141 g (91,8 % der Theorie),
   [α]_{D}: + 161,1° (c = 1,928, Methanol) erhalten.
   Das Salz wird zweimal aus Isopropanol/Diisopropylether (4 : 1) umkristallisiert.
   Ausbeute: 112,5 g,
   [α]_{D}: + 215,7° (c = 1,1, Methanol).
   Freisetzung der Säure: 17 g (49,3 mmol) dieser Kristalle werden in 100 ml Eiswasser suspendiert und mit 3 ml kozentrierter Schwefelsäure angesäuert. Dann wird dreimal mit je 100 ml tert.-Butylmethylether extrahiert, die organischen Phasen über MgSO₄ getrocknet und eingeengt.
   Rohausbeute: 13,2 g,
   Schmelzpunkt: 79-81° (aus Diisopropylether),
   [α]^{D} = + 254° (c = 0,85, CH₂Cl₂)
H. (1S,6R)-1-Methoxycarbonylamino-8-azabicyclo[4.3.0]nona-2,4-dien-8-carbonsäure-methyl-ester ((3aS,7aR)-3a-Methoxycarbonylamino-1,2,3,7a-tetrahydro-isoindol-2-carbonsäure-methylester)
   Analog zu Stufe E werden 13 g roher (1S,6S)-8-Azabicyclo[4.3.0]nona-2,4-dien-1,8-dicarbonsäure-8-methylester mit 5 g (50 mmol) Triethylamin, 3,2 g Methanol, 13,7 g (55 mmol) Diphenylphosphorylazid in 160 ml absolutem Toluol umgesetzt und entsprechend aufgearbeitet.
   Rohausbeute: 11,2 g
I. (3aS,7aR)-1,2,3,7a-Tetrahydro-isoindol-3a-ylamin ((1S,6R)-1-Amino-8-azabicyclo[4.3.0]nona-2,4-dien)
   Analog Stufe F werden 11 g roher (1S,6R)-1-Methoxycarbonylamino-8-azabicyclo[4.3.0]-nona-2,4-dien-8-carbonsäuremethylester mit 42 g Ba(OH)₂·8 H₂O in 150 ml Wasser verseift und entsprechend aufgearbeitet.
   Ausbeute: 3 g (44,6 % der Theorie bezogen auf Stufe G),
   Siedepunkt: 70°C/ 0,1 mbar,
   [α]_{D} = + 235,9° (c = 1,14, Methanol).
J. (1R,6R)-8-Azabicyclo[4.3.0]nona-2,4-dien-1,8-dicarbonsäure-8-methylester ((3aR,7aR)-1,2,3,7a-Tetrahydro-isoindol-2,3a-dicarbonsäure-2-methylester)
   Analog Stufe G (Methode 1) wird die Racematspaltung mit S-(-)-Phenylethylamin durchgeführt und (1R,6R)-8-Azabicyclo[4.3.0]nona-2,4-dien-1,8-dicarbonsäure-8-methylester erhalten, [α]_{D} = - 233,6° (c = 0,6, CH₂Cl₂).
K. (1R,6S)-1-Methoxycarbonylamino-8-azabicyclo[4.3.0]nona-2,4-dien-8-carbonsäuremethyl-ester ((3aR,7aS)-3a-Methoxycarbonylamino-1,2,3,7a-tetrahydro-isoindol-2-carbonsäuremethylester)
   Man setzt das Produkt aus Stufe J analog Stufe H um und erhält (1R,6S)-1-Methoxycarbonylamino-8-azabicyclo[4.3.0]nona-2,4-dien-8-carbonsäuremethylester, der als Rohprodukt weiter umgesetzt wird.
L. (3aR,7aS)-1,2,3,7a Tetrahydro-isoindol-3a-ylamin ((1R,6S)-1-Amino-8-azabicyclo[4.3.0]nona-2,4-dien)
   Das in Stufe K erhaltene Produkt wird analog den Angaben in Stufe F umgesetzt, [α]_{D}: - 224° (c = 0,8, Methanol).
M. 8-Azabicyclo[4.3.0]nona-2,4-dien-1-carboxamid-8-carbonsäuremethylester (1,2,3,7a-Tetrahydro-isoindol-3a-carboxamid-2-carbonsäuremethylester)
   Man legt 4,5 g (20 mmol) 8-Azabicyclo[4.3.0]nona-2,4-dien-1,8-dicarbonsäure-8-methylester in 20 ml absolutem CH₂Cl₂ vor und setzt 2,2 g (22 mmol) Triethylamin hinzu. Man kühlt auf -20°C, tropft 2,6 g (25 mmol) Chlorameisensäureethylester hinzu und rührt eine Stunde bei -20°C. Dann tropft man bei dieser Temperatur 20 ml 25%iger wäßrige Ammoniaklösung hinzu, läßt auf Raumtemperatur kommen und rührt noch 1 Stunde nach. Dann extrahiert man mehrfach mit CH₂Cl₂, trocknet über MgSO₄ und engt ein. Das Produkt kristallisiert.
   Ausbeute: 4,4 g (99 % der Theorie),
   Schmelzpunkt: 117-120°C (aus Toluol).
N. 1-Amino-8-azabicyclo[4.3.0]nona-2,4-dien-8-carbonsäuremethylester (3a-Amino-1,2,3,7a-tetrahydro-isoindol-2-carbonsäuremethylester)
   Man erhitzt 4,3 g (19,4 mmol) 8-Azabicyclo[4.3.0]nona-2,4-dien-1-carboxamid-8-carbonsäuremethylester mit 7,9 g (20,2 mmol) I-Hydroxy-I-tosyloxyiodbenzol in 100 ml absolutem Acetonitril 3 Stunden unter Rückfluß. Man engt die Lösung ein, nimmt in 100 ml CHCl₃ auf, wäscht mit 15%iger KOH-Lösung, trocknet über MgSO₄, engt ein und destilliert im Hochvakuum.
   Ausbeute: 1,5 g (40 % der Theorie),
   Siedepunkt: 122-125°C/ 0,07 mbar.
O. 1,2,3,7a-Tetrahydro-isoindol-3a-ylamin (1-Amino-8-azabicyclo[4.3.0]nona-2,4-dien)
   Analog Stufe F werden 1,4 g (7,2 mmol) 1-Amino-8-azabicyclo[4.3.0]nona-2,4-dien-8-carbonsäuremethylester mit 4 g Ba(OH)₂·8H₂O in 20 ml Wasser verseift und entsprechend aufgearbeitet.
   Ausbeute: 0,6 g (61 % der Theorie),
   Siedepunkt: 65°C/ 0,1 mbar.

### Herstellung der Wirkstoffe

### Beispiel 1

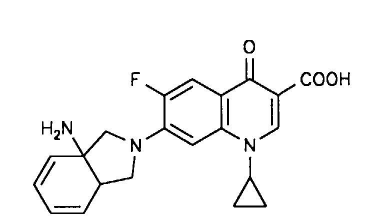

265 mg (1 mmol) 1-Cyclopropyl-6,7-difluor-1,4-dihydro-4-oxo-3-chinolincarbonsäure werden in einer Mischung aus 4 ml Acetonitril und 2 ml Dimethylformamid mit 170 mg (1,5 mmol) 1,4-Diazabicyclo[2.2.2]octan und 150 mg (1,1 mmol) 1,2,3,7a-Tetrahydro-isoindol-3a-ylamin 1 Stunde unter Rückfluß erhitzt. Der ausgefallene Niederschlag wird abgesaugt, mit 30 ml Wasser gewaschen und getrocknet.

Ausbeute: 288 mg (75,6 % der Theorie) 7-(3a-Amino-1,2,3,7a-tetrahydro-isoindol-2-yl)-1-cyclopropyl-6-fluor-1,4-dihydro-4-oxo-3-chinolincarbonsäure,
Schmelzpunkt: 272-274°C (unter Zersetzung).

### Beispiel 2

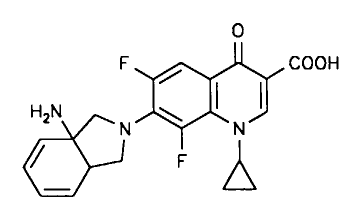

Unter entsprechenden Bedingungen wie in Beispiel 1 erhält man mit 1-Cyclopropyl-6,7,8-trifluor-1,4-dihydro-4-oxo-3-chinolincarbonsäure in 85 %-iger Ausbeute 7-(3a-Amino-1,2,3,7a-tetrahydro-isoindol-2-yl)-1-cyclopropyl-6,8-difluor-1,4-dihydro-4-oxo-3-chinolincarbonsäure vom Schmelzpunkt: 232-233°C (unter Zersetzung).

### Beispiel 3

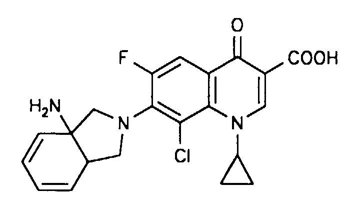

Unter entsprechenden Bedingungen wie in Beispiel 1 erhält man mit 8-Chlor-1-cyclopropyl-6,7-difluor-1,4-dihydro-4-oxo-3-chinolincarbonsäure in 58 %-iger Ausbeute 7-(3a-Amino-1,2,3,7a-tetrahydro-isoindol-2-yl)-8-chlor-1-cyclopropyl-6-fluor-1,4-dihydro-4-oxo-3-chinolincarbonsäure vom Schmelzpunkt: 179-182°C (unter Zersetzung).

### Beispiel 4

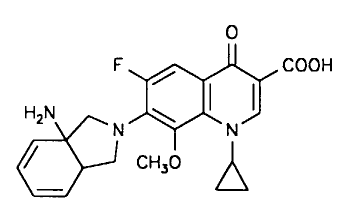

295 mg (1 mmol) 1-Cyclopropyl-6,7-difluor-1,4-dihydro-8-methoxy-4-oxo-3-chinolincarbonsäure werden in einer Mischung aus 4 ml Acetonitril und 2 ml Dimethylformamid mit 330 mg (2,4 mmol) 1,2,3,7a-Tetrahydro-isoindol-3a-ylamin 1 Stunde unter Rückfluß erhitzt. Die Mischung wird eingeengt, der Rückstand mit 40 ml Wasser verrüht, der langsam ausfallende Niederschlag abgesaugt, mit Wasser gewaschen und bei 60°C im Hochvakuum getrocknet.

Ausbeute: 175 mg (43 % der Theorie) 7-(3a-Amino-1,2,3,7a-tetrahydro-isoindol-2-yl)-1-cyclopropyl-6-fluor-1,4-dihydro-8-methoxy-4-oxo-3-chinolincarbonsäure,
Schmelzpunkt: 195-196°C (unter Zersetzung).

### Beispiel 5

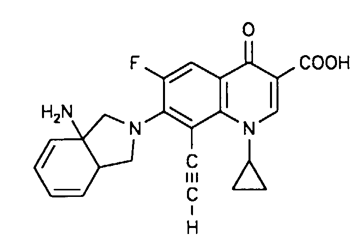

289 mg (1 mmol) 1-Cyclopropyl-8-ethinyl-6,7-difluor-1,4-dihydro-4-oxo-3-chinolincarbonsäure werden in einer Mischung aus 4 ml Acetonitril und 2 ml Dimethylformamid mit 170 mg (1,5 mmol) 1,4-Diazabicyclo[2.2.2]octan und 150 mg (1,1 mmol) 1,2,3,7a-Tetrahydro-isoindol-3a-ylamin 1 Stunde unter Rückfluß erhitzt. Die Mischung wird eingeengt, der Rückstand mit Wasser verrührt (pH = 8), mit verdünnter Salzsäure auf pH = 7 eingestellt, der ausgefallene Niederschlag wird abgesaugt, mit Wasser gewaschen und getrocknet.

Ausbeute: 382 mg (94 % der Theorie) 7-(3a-Amino-1,2,3,7a-tetrahydro-isoindol-2-yl)-1-cyclopropyl-8-ethinyl-6-fluor-1,4-dihydro-4-oxo-3-chinolincarbonsäure,
Schmelzpunkt: 176-177°C (unter Zersetzung).

### Beispiel 6

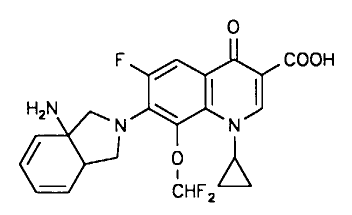

Unter entsprechenden Bedingungen wie in Beispiel 5 erhält man mit 1-Cyclopropyl-8-difluormethoxy-6,7-difluor-1,4-dihydro-4-oxo-3-chinolincarbonsäure in 66 %-iger Ausbeute 7-(3a-Amino-1,2,3,7a-tetrahydro-isoindol-2-yl)-1-cyclopropyl-8-difluormethoxy-6-fluor-1,4-dihydro-4-oxo-3-chinolincarbonsäure vom Schmelzpunkt: 215-217°C (unter Zersetzung).

### Beispiel 7

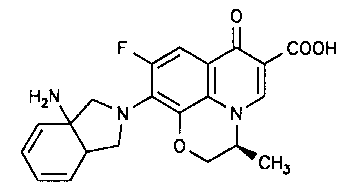

Unter entsprechenden Bedingungen wie in Beispiel 1 erhält man mit (S)-9,10-Difluor-2,3-dihydro-3-methyl-7-oxo-7H-pyrido[1,2,3-d,e][1,4]benzoxazin-6-carbonsäure in 45 %-iger Ausbeute 10-(3a-Amino-1,2,3,7a-tetrahydro-isoindol-2-yl)-9-fluor-2,3-dihydro-3(S)-methyl-7-oxo-7H-pyrido[1,2,3-d,e][1,4]benzoxazin-6-carbonsäure vom Schmelzpunkt: 242-243°C (unter Zersetzung).

### Beispiel 8

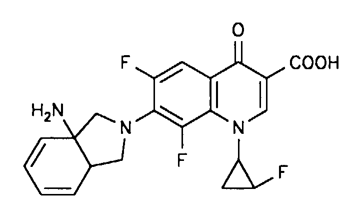

Unter entsprechenden Bedingungen wie in Beispiel 1 erhält man mit racemischem 6,7,8-Trifluor-1-(cis-2-fluorcyclopropyl)-1,4-dihydro-4-oxo-3-chinolincarbonsäure in 66 %-iger Ausbeute 7-(3a-Amino-1,2,3,7a-tetrahydro-isoindol-2-yl)-6,8-difluor-1-(cis-2-fluorcyclopropyl)-1,4-dihydro-4-oxo-3-chinolincarbonsäure vom Schmelzpunkt: 210-211°C (unter Zersetzung).

### Beispiel 9

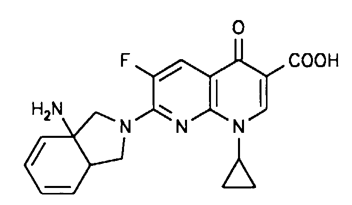

283 mg (1 mmol) 7-Chlor-1-cyclopropyl-6-fluor-1,4-dihydro-4-oxo-1,8-naphthyridin-3-carbonsäure werden in 6 ml Acetonitril bei 25°C mit 270 mg (2 mmol) 1,2,3,7a-Tetrahydro-isoindol-3a-ylamin versetzt und 1 Stunde bei 50°C gerührt. Man kühlt die Suspension im Eisbad, saugt den Niederschlag ab, wäscht mit Acetonitril und verrührt mit Wasser und trocknet bei 80°C/0,1 mbar.

Ausbeute: 262 mg (67 % der Theorie) 7-(3a-Amino-1,2,3,7a-tetrahydro-isoindol-2-yl)-1-cyclopropyl-6-fluor-1,4-dihydro-4-oxo-1,8-naphthyridin-3-carbonsäure,
Schmelzpunkt: 239-240°C (unter Zersetzung).

### Beispiel 10

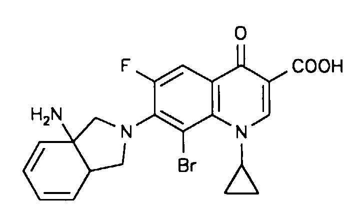

Unter entsprechenden Bedingungen wie in Beispiel 1 erhält man mit 8-Brom-1-cyclopropyl-6,7-difluor-1,4-dihydro-4-oxo-3-chinolincarbonsäure ein Reaktionsgemisch, aus dem 7-(3a-Amino-1,2,3,7a-tetrahydro-isoindol-2-yl)-8-brom-1-cyclopropyl-6-fluor-1,4-dihydro-4-oxo-3-chinolincarbonsäure durch Chromatographie an Kieselgel (Laufmittel: Dichlormethan/Methanol/17%-Ammoniak = 30:8:1) isoliert wurde;
Schmelzpunkt: 200-201°C (unter Zersetzung).

### Beispiel 11

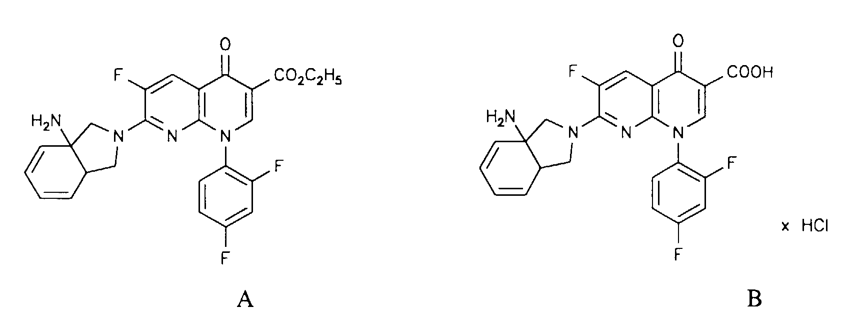

A. 358 mg (1 mmol) 7-Chlor-1-(2,4-difluorphenyl)-6-fluor-1,4-dihydro-4-oxo-1,8-naphthyridin-3-carbonsäureethylester werden in 6 ml Acetonitril mit 202 mg (1,5 mmol) 1,2,3,7a-Tetrahydro-isoindol-3a-ylamin versetzt und 2 Stunden bei 30°C gerührt. Man saugt den Niederschlag ab, wäscht mit Acetonitril, trocknet bei 90°C/0,1 mbar (Rohausbeute: 255 mg) und reinigt durch Chromatographie an 15 g Kielselgel (Laufmittel: Dichlormethan/Methanol/17 % Ammoniak 30:8:1).
   Ausbeute: 86 mg (18 % der Theorie) 7-(3a-Amino-1,2,3,7a-tetrahydro-isoindol-2-yl)-6-fluor-1-(2,4-difluorphenyl)-1,4-dihydro-4-oxo-1,8-naphthyridin-3-carbonsäureethylester,
   Schmelzpunkt: 202-207°C (unter Zersetzung).
B. 80 mg des Produktes aus Stufe A werden in einer Mischung aus 1 ml Essigsäure und 0,75 ml halbkonzentrierter Salzsäure 2 Stunden unter Rückfluß erhitzt. Die Mischung wird eingeengt, der Rückstand mit wenig Wasser verrührt, der Niederschlag abgesaugt, mit Wasser gewaschen und bei 100°C im Hochvakuum getrocknet.
   Ausbeute: 37 mg (45 % der Theorie) 7-(3a-Amino-1,2,3,7a-tetrahydro-isoindol-2-yl)-6-fluor-1-(2,4-difluorphenyl)-1,4-dihydro-4-oxo-1,8-naphthyridin-3-carbonsäure-Hydrochlorid,
   Schmelzpunkt: 208-210°C (unter Zersetzung).

### Beispiel 12

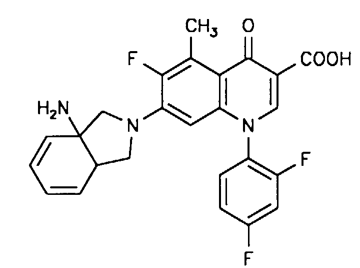

Unter entsprechenden Bedingungen wie in Beispiel 4 erhält man mit 1-(2,4-Difluorphenyl)-6,7-difluor-1,4-dihydro-5-methyl-4-oxo-3-chinolincarbonsäure in 89 %-iger Ausbeute 7-(3a-Amino-1,2,3,7a-tetrahydro-isoindol-2-yl)-1-(2,4-difluorphenyl)-6-fluor-1,4-dihydro-5-methyl-4-oxo-3-chinolincarbonsäure,
Schmelzpunkt: 157-159°C (unter Zersetzung).

### Beispiel 13

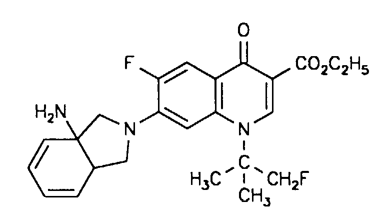

Unter entsprechenden Bedingungen wie in Beispiel 1 erhält man mit 6,7-Difluor-1-(fluor-tert.-butyl)-1,4-dihydro-4-oxo-3-chinolincarbonsäureethylester ein Reaktionsgemisch, aus welchem 7-(3a-Amino-1,2,3,7a-tetrahydro-isoindol-2-yl)-6-fluor-1-(fluor-tert.-butyl)-1,4-dihydro-4-oxo-3-chinolincarbonsäureethylester durch Chromatographie an Kieslegel (Dichlormethan/Methanol = 95:5) isoliert wurde;
Schmelzpunkt: 219-220°C (unter Zersetzung).

### Beispiel 14

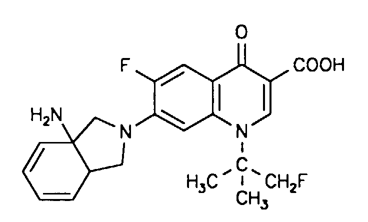

Unter entsprechenden Bedingungen wie in Beispiel 4 erhält man mit 6,7-Difluor-1-(fluor-tert.butyl)-1,4-dihydro-4-oxo-3-chinolincarbonsäure in 78 %-iger Ausbeute 7-(3a-Amino-1,2,3,7a-tetrahydro-isoindol-2-yl)-6-fluor-1-(fluor-tert.butyl)-1,4-dihydro-4-oxo-3-chinolincarbonsäure vom Schmelzpunkt: 229-231°C (unter Zersetzung).

### Beispiel 15

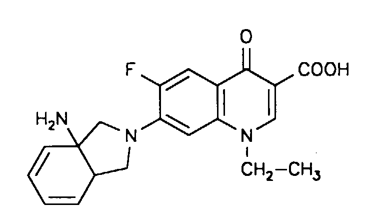

Unter entsprechenden Bedingungen wie in Beispiel 1 erhält man mit 1-Ethyl-6,7-difluor-1,4-dihydro-4-oxo-3-chinolincarbonsäure in 63 %-iger Ausbeute 7-(3a-Amino-1,2,3,7a-tetrahydro-isoindol-2-yl)-1-ethyl-6-fluor-1,4-dihydro-4-oxo-3-chinolincarbonsäure vom Schmelzpunkt: 229°C (unter Zersetzung).

### Beispiel 16

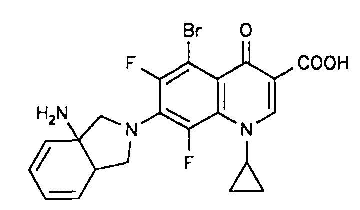

Unter entsprechenden Bedingungen wie in Beispiel 1 erhält man mit 5-Brom-1-cyclopropyl-6,7,8-trifluor-1,4-dihydro-4-oxo-3-chinolincarbonsäure in 71 %-iger Ausbeute 7-(3a-Amino-1,2,3,7a-tetrahydro-isoindol-2-yl)-5-brom-1-cyclopropyl-6,8-difluor-1,4-dihydro-4-oxo-3-chinolincarbonsäure vom Schmelzpunkt: 278-280°C (unter Zersetzung).

### Beispiel 17

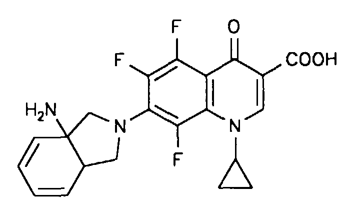

Unter entsprechenden Bedingungen wie in Beispiel 1 erhält man mit 1-Cyclopropyl-5,6,7,8-tetrafluor-1,4-dihydro-4-oxo-3-chinolincarbonsäure in 70 %-iger Ausbeute 7-(3a-Amino-1,2,3,7a-tetrahydro-isoindol-2-yl)-1-cyclopropyl-5,6,8-trifluor-1,4-dihydro-4-oxo-3-chinolincarbonsäure,
Schmelzpunkt: 244-245°C (unter Zersetzung).

### Beispiel 18

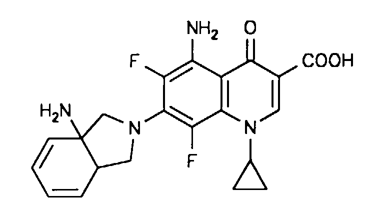

In eine Lösung von 50 mg (0,12 mmol) 7-(3a-Amino-1,2,3,7a-tetrahydro-isoindol-2-yl)-1-cyclopropyl-5,6,8-trifluor-1,4-dihydro-4-oxo-3-chinolincarbonsäure in 5 ml Dimethylsulfoxid wird während 14 Stunden bei 110°C bis 120°C ein Ammoniakstrom eingeleitet. Die Mischung wird eingedampft und der Rückstand mit 8 ml Ethanol verrührt. Der ungelöste Niederschlag wird abgesaugt, mit Ethanol gewaschen, bei 60°C im Hochvakuum getrocknet (27 mg Rohprodukt) und chromatographisch (Kieselgel, Laufmittel: Dichlormethan/Methanol = 95 : 5) gereinigt.

Ausbeute: 18 mg 5-Amino-7-(3a-amino-1,2,3,7a-tetrahydro-isoindol-2-yl)-1-cyclopropyl-6,8-difluor-1,4-dihydro-4-oxo-3-chinolincarbonsäure,
Schmelzpunkt: 194-195°C (unter Zersetzung).

### Beispiel 19

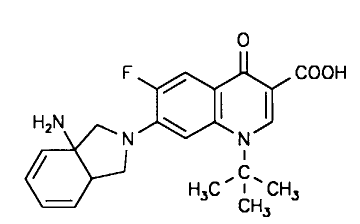

Unter entsprechenden Bedingungen wie in Beispiel 5 erhält man mit 1-tert.-Butyl-6,7-difluor-1,4-dihydro-4-oxo-3-chinolincarbonsäure in 69 %-iger Ausbeute 7-(3a-Amino-1,2,3,7a-tetrahydro-isoindol-2-yl)-1-tert.-butyl-6-fluor-1,4-dihydro-4-oxo-3-chinolincarbonsäure vom Schmelzpunkt: 228-230°C (unter Zersetzung).

### Beispiel 20

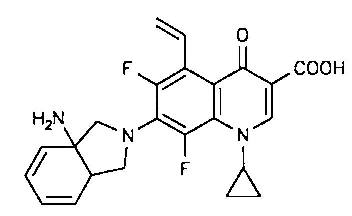

Unter entsprechenden Bedingungen wie in Beispiel 4 erhält man mit 1-Cyclopropyl-6,7,8-trifluor-1,4-dihydro-4-oxo-5-vinyl-3-chinolincarbonsäure in 75 %-iger Ausbeute 7-(3a-Amino-1,2,3,7a-tetrahydro-isoindol-2-yl)-1-cyclopropyl-6-fluor-1,4-dihydro-4-oxo-5-vinyl-3-chinolincarbonsäure,
Schmelzpunkt: 227-228°C (unter Zersetzung).

### Beispiel 21

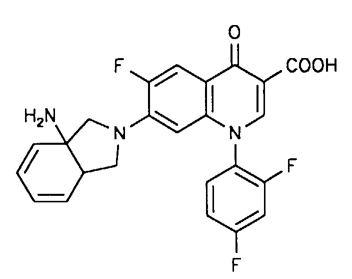

Unter entsprechenden Bedingungen wie in Beispiel 4 erhält man mit 1-(2,4-Difluorphenyl)-6,7-difluor-1,4-dihydro-4-oxo-3-chinolincarbonsäure in 77 %-iger Ausbeute 7-(3a-Amino-1,2,3,7a-tetrahydro-isoindol-2-yl)-1-(2,4-difluorphenyl)-6-fluor-1,4-dihydro-4-oxo-3-chinolincarbonsäure vom Schmelzpunkt: 253-254°C (unter Zersetzung).

### Beispiel 22

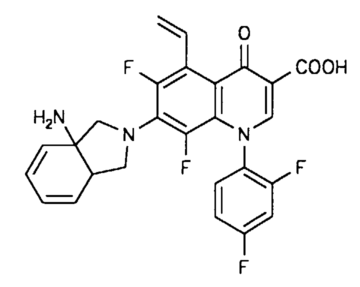

Unter entsprechenden Bedingungen wie in Beispiel 4 erhält man mit 1-(2,4-Difluorphenyl)-6,7,8-trifluor-1,4-dihydro-4-oxo-5-vinyl-3-chinolincarbonsäure in 96 %-iger Ausbeute 7-(3a-Amino-1,2,3,7a-tetrahydro-isoindol-2-yl)-1-(2,4-difluorphenyl)-6,8-difluor-1,4-dihydro-4-oxo-5-vinyl-3-chinolincarbonsäure vom Schmelzpunkt: 215-216°C (unter Zersetzung).

### Beispiel 23

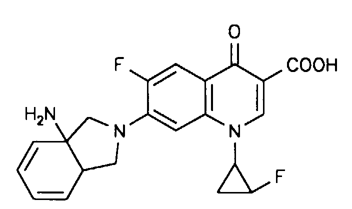

Unter entsprechenden Bedingungen wie in Beispiel 1 erhält man mit racemischem 6,7-Difluor-1-(cis-2-fluorcyclopropyl)-1,4-dihydro-4-oxo-3-chinolincarbonsäure in 55 %-iger Ausbeute 7-(3a-Amino-1,2,3,7a-tetrahydro-isoindol-2yl)-6-fluor-1-(cis-2-fluorcyclopropyl)-1,4-dihydro-4-oxo-3-chinolincarbonsäure vom Schmelzpunkt: 238-239°C (unter Zersetzung).

### Beispiel 24

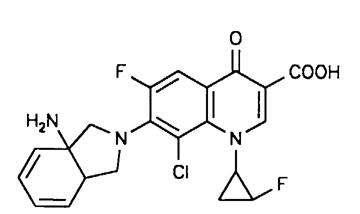

Unter entsprechenden Bedingungen wie in Beispiel 1 erhält man mit racemischem 8-Chlor-6,7-difluor-1-(cis-2-fluorcyclopropyl)-1,4-dihydro-4-oxo-3-chinolincarbonsäure in 52 %-iger Ausbeute 7-(3a-Amino-1,2,3,7a-tetrahydro-isoindol-2-yl)-8-chlor-6-fluor-1-(cis-2-fluorcyclopropyl)-1,4-dihydro-4-oxo-3-chinolincarbonsäure vom Schmelzpunkt: 196-198°C (unter Zersetzung) (nach chromatographischer Reinigung mit Dichlormethan/Methanol (95:5) an Kieselgel).

### Beispiel 25

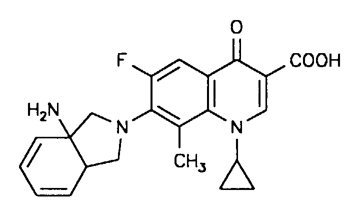

410 mg (1 mmol) 1-Cyclopropyl-6,7-difluor-1,4-dihydro-8-methyl-4-oxo-3-chinolincarbonsäure B(O-CO-CH₃)₂-Chelat werden in 8 ml Acetonitril unter Stickstoff mit 224 mg (2 mmol) 1,4-Diazabicyclo[2.2.2]octan und 272 mg (2 mmol) 1,2,3,7a-Tetrahydro-isoindol-3a-ylamin 15 Stunden auf 60-70°C erhitzt. Die Mischung wird im Vakuum eingeengt und der Rückstand mit einer Mischung aus 4 ml Aceton und 0,5 ml konzentrierter Salzsäure versetzt und während 30 Minuten im Ultraschallbad behandelt. Man engt ein, nimmt den Rückstand in Wasser auf (pH 3), saugt die ausgefallene 1-Cyclopropyl-6,7-difluor-1,4-dihydro-8-methyl-4-oxo-3-chinolincarbonsäure (90 mg) ab und stellt die Mutterlauge mit 5 %-iger Natriumbicarbonat-Lösung auf pH 7,5. Man extrahiert mit Dichlormethan, trocknet mit Natriumsulfat und engt ein.

Ausbeute: 61 mg (15 % der Theorie) 7-(3a-Amino-1,2,3,7a-tetrahydro-isoindol-2-yl)-1-cyclopropyl-6-fluor-1,4-dihydro-8-methyl-4-oxo-3-chinolincarbonsäure,
Schmelzpunkt: 201-203°C (unter Zersetzung).

### Beispiel 26

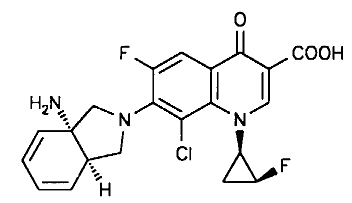

317 mg (1 mmol) 8-Chlor-6,7-difluor-1-[(1R,2S)-2-fluorcyclopropyl]-1,4-dihydro-4-oxo-3-chinolincarbonsäure werden in einer Mischung aus 4 ml Acetonitril und 2 ml Dimethylformamid mit 187 mg (1,67 mmol) 1,4-Diazabicyclo[2.2.2]octan und 165 mg (1,2 mmol) (3aS,7aR)-1,2,3,7a-Tetrahydro-isoindol-3a-ylamin 1 Stunde unter Rückfluß erhitzt. Die Lösung bleibt über Nacht im Kühlschrank stehen. Der ausgefallene Niederschlag wird abgesaugt, mit 30 ml Wasser gewaschen und getrocknet.

Ausbeute: 290 mg (67 % der Theorie) 7-([3aS,7aR]3a-Amino-1,2,3,7a-tetrahydro-isoindol-2-yl)-8-chlor-1-[(1R,2S)-2-fluorcyclopropyl]-6-fluor-1,4-dihydro-4-oxo-3-chinolincarbonsäure,
Schmelzpunkt: 206-207°C (unter Zersetzung),
[α]_{D}: + 2,5° (c = 0,5, CHCl₃), unterschiedliche Ergebnisse bei Drehwertsmessung; Strukturaufklärung durch Röntgenstrukturanalyse.

217 mg (0,5 mmol) 7-[(3aS, 7aR) 3a-Amino-1,2,3,7a-tetrahydro-isoindol-2-yl]-8-chlor-1-[(1R,2S)-2-fluorcyclopropyl]-6-fluor-1,4-dihydro-4-oxo-3-chinolinecarbosäure werden in eine Mischung aus 5 ml Wasser und 0,5 ml 1n Salzsäure gelöst und diese Lösung lyophilisiert. Das 7-[(3aS, 7aR) 3a-Amino-1,2,3,7a-tetrahydro-isoindol-2-yl]-8-chlor-1-[(1R, 2S)-2-fluorcyclopropyl]-6-fluor-1,4-dihydro-4-oxo-3-chinolincarbonsäure Hydrochlorid wird in quantitativer Ausbeute isoliert.

Unter ähnlichen Bedingungen werden auch das Mesylat und das Tosylat hergestellt.

### Beispiel 27

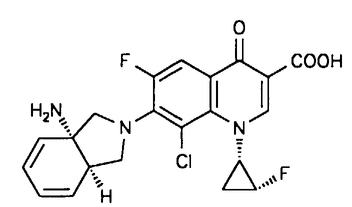

Unter entsprechenden Bedingungen wie in Beispiel 26 erhält man mit 8-Chlor-6,7-difluor-1-[(1S,2R)-2-fluorcyclopropyl]-1,4-dihydro-4-oxo-3-chinolincarbonsäure in 71 %-iger Ausbeute 7-([3aS,7aR]3a-Amino-1,2,3,7a-tetrahydro-isoindol-2-yl)-8-chlor-6-fluor-1-[(1S,2R)-2-fluorcyclopropyl]-1,4-dihydro-4-oxo-3-chinolincarbonsäure vom Schmelzpunkt: 170-174°C (unter Zersetzung),
[α]_{D}: + 215° (c = 0,5, CHCl₃).

### Beispiel 28

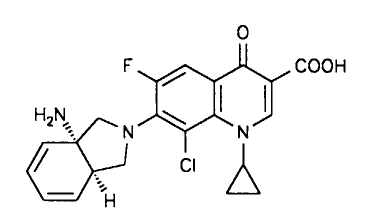

Unter entsprechenden Bedingungen wie in Beispiel 26 erhält man mit 8-Chlor-1-cyclopropyl-6,7-difluor-1,4-dihydro-4-oxo-3-chinolincarbonsaure in 86 %-iger Ausbeute 7-([3aS,7aR]3a-Amino-1,2,3,7a-tetrahydro-isoindol-2-yl)-8-chlor-1-cyclo propyl-6-fluor-1,4-dihydro-4-oxo-3-chinolincarbonsäure vom Schmelzpunkt: 169-170°C (unter Zersetzung), [α]_{D}: + 116° (c = 0,4, CHCl₃).

### Beispiel 29

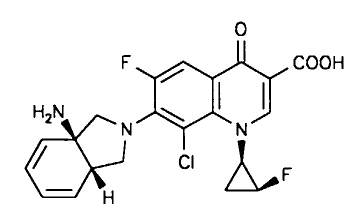

317 mg (1 mmol) 8-Chlor-6,7-difluor-1-[(1R,2S)-2-fluorcyclopropyl]-1,4-dihydro-4-oxo-3-chinolincarbonsäure werden in einer Mischung aus 4 ml Acetonitril und 2 ml Dimethylformamid mit 187 mg (1,67 mmol) 1,4-Diazabicyclo[2.2.2]octan und 165 mg (1,2 mmol) (3aR,7aS)-1,2,3,7a-Tetrahydro-isoindol-3a-ylamin 1 Stunde unter Rückfluß erhitzt. Die Lösung bleibt über Nacht im Kühlschrank stehen. Der ausgefallene Niederschlag wird abgesaugt, mit 30 ml Wasser gewaschen und getrocknet.

Ausbeute: 235 mg (54 % der Theorie) 7-([3aR,7aS]3a-Amino-1,2,3,7a-tetrahydro-isoindol-2-yl)-8-chlor-1-[(1R,2S)-2-fluorcyclopropyl]-6-fluor-1,4-dihydro-4-oxo-3-chinolincarbonsäure,
Schmelzpunkt: 182-183°C (unter Zersetzung),
[α]_{D}: - 245° (c = 0,5, CHCl₃).

### Beispiel 30

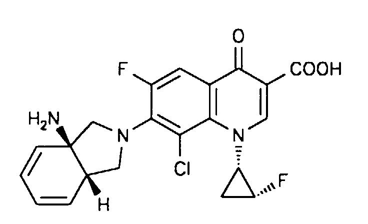

Unter entsprechenden Bedingungen wie in Beispiel 29 erhält man mit 8-Chlor-6,7-difluor-1-[(1S,2R)-2-fluorcyclopropyl]-1,4-dihydro-4-oxo-3-chinolincarbonsäure in 71 %-iger Ausbeute 7-([3aR,7aS]3a-Amino-1,2,3,7a-tetrahydro-isoindol-2-yl)-8-chlor-6-fluor-1-[(1S,2R)-2-fluorcyclopropyl]-1,4-dihydro-4-oxo-3-chinolincarbonsäure vom Schmelzpunkt: 195-197°C (unter Zersetzung),
[α]_{D}: - 6,4° (c = 0,5, CHCl₃), schwankende Ergebnisse bei der Drehwertsmessung.

### Beispiel 31

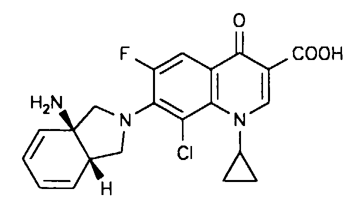

Unter entsprechenden Bedingungen wie in Beispiel 29 erhält man mit 8-Chlor-6,7-difluor-1-cyclopropyl-1,4-dihydro-4-oxo-3-chinolincarbonsäure in 90 %-iger Ausbeute 7-([3aR,7aS]3a-Amino-1,2,3,7a-tetrahydro-isoindol-2-yl)-8-chlor-6-fluor-1-cyclopropyl-1,4-dihydro-4-oxo-3-chinolincarbonsäure vom Schmelzpunkt: 169-170°C (unter Zersetzung), [α]_{D}: - 119° (c = 0,4, CHCl₃).

## Patentansprüche

1. Verbindungen der Formel (I)
T-Q (I),
in welcher
Q einen Rest der Formeln 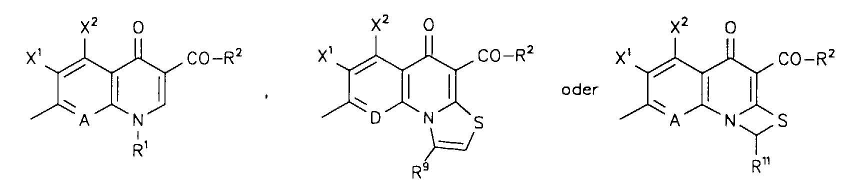
bedeutet, worin
R¹ für gegebenenfalls durch Halogen oder Hydroxy ein- oder zweifach substituiertes Alkyl mit 1 bis 4 Kohlenstoffatomen, Alkenyl mit 2 bis 4 Kohlenstoffatomen, gegebenenfalls durch 1 oder 2 Fluoratome substituiertes Cycloalkyl mit 3 bis 6 Kohlenstoffatomen, Bicyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl,
R² für Hydroxy, gegebenenfalls durch Hydroxy, Methoxy, Amino, Dimethylamino substituiertes Alkoxy mit 1 bis 3 Kohlenstoffatomen, Benzyloxy oder (5-Methyl-2-oxo-1,3-dioxol-4-yl)-methyl-oxy, Acetoxymethyloxy, Pivaloyloxymethyloxy, 5-Indanyloxy, Phthalidinyloxy, 3-Acetoxy-2-oxo-butyloxy, Nitromethyl oder Dialkoxycarbonylmethyl mit 1 bis 2 Kohlenstoffatomen in jedem Alkylteil,
R⁹ für Wasserstoff oder gegebenenfalls durch Methoxy, Hydroxy oder Halogen substituiertes Alkyl mit 1 bis 3 Kohlenstoffatomen,
R¹¹ für Wasserstoff, CH₃ oder CH₂F,
X¹ für Halogen oder Nitro,
X² für Wasserstoff, Halogen, Amino, Hydroxy, Methoxy, Mercapto, Methyl, Halogenmethyl oder Vinyl,
A für N oder C-R⁷ steht, worin
R⁷ für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*S-CH₂-CH-CH₃, -*CH₂-CH₂-CH-CH₃ oder -*O-CH₂-N-R⁸, wobei das mit markierte Atom mit dem Kohlenstoffatom von A verknüpft ist und worin
R⁸ Wasserstoff, Methyl oder Formyl bedeutet, bilden kann, und
D für N oder C-R¹⁰ steht, worin
R¹⁰ für Wasserstoff, Halogen, CF₃, OCH₃, OCHF₂ oder CH₃ steht oder auch gemeinsam mit R⁹ eine Brücke der Struktur -*O-CH₂-, -*NH-CH₂-, -*N(CH₃)-CH₂-, -*N(C₂H₅)-CH₂-, -*N(c-C₃H₅)-CH₂- oder -*S-CH₂- bilden kann, wobei das mit * markierte Atom mit dem Kohlenstoffatom von D verknüpft ist, und
T einen Rest der Formel 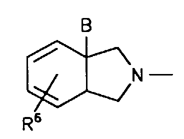 bedeutet, worin
B für (CH₂)ₘ-NR³R⁴ oder (CH₂)ₘ-OR⁵ steht, worin
m für 0 oder 1,
R³ für Wasserstoff Methyl oder Alkoxycarbonyl mit 1 bis 4 Kohlenstoffatomen im Alkylteil,
R⁴ für Wasserstoff oder Methyl und
R⁵ für Wasserstoff oder Methyl und
R⁶ für Wasserstoff oder Methyl steht,
und deren pharmazeutisch verwendbaren Hydrate und Säureadditionssalze sowie die Alkali-, Erdalkali-, Silber- und Guanidiniumsalze der zugrundeliegenden Carbonsäuren.

2. Verbindungen der Formel (I) gemäß Anspruch 1,
in welcher
Q einen Rest der Formeln
bedeutet, worin
R¹ für gegebenenfalls durch Halogen ein- oder zweifach substituiertes Alkyl mit 1 bis 4 Kohlenstoffatomen, Alkenyl mit 2 bis 3 Kohlenstoffatomen, gegebenenfalls durch 1 Fluoratom substituiertes Cycloalkyl mit 3 oder 4 Kohlenstoffatomen, Bicyclo[1.1.1]pent-1-yl, 1,1-Dimethylpropargyl, 3-Oxetanyl, Methylamino, gegebenenfalls durch Fluor, Amino oder Hydroxy ein- oder zweifach substituiertes Phenyl,
R² für Hydroxy, Alkoxy mit 1 bis 2 Kohlenstoffatomen, Benzyloxy oder (5-Methyl-2-oxo-1,3-dioxol-4-yl)-methoxy,
R⁹ für Wasserstoff oder gegebenenfalls durch Fluor ein- bis dreifach substituiertes Alkyl mit 1 bis 2 Kohlenstoffatomen,
X¹ für Fluor oder Chlor,
X² für Wasserstoff, Halogen, Amino, Methyl, Trifluormethyl oder Vinyl,
A für N oder C-R⁷ steht, worin
R⁷ für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*CH₂-CH₂-CH-CH₃ oder -*O-CH₂-N-R⁸, wobei das mit * markierte Atom mit dem Kohlenstoffatom von A verknüpft ist und worin
R⁸ Wasserstoff oder Methyl bedeutet, bilden kann, und
D für N oder C-R¹⁰ steht, worin
R¹⁰ für Wasserstoff, Fluor, Chlor, CF₃, OCH₃ oder CH₃ steht oder auch gemeinsam mit R⁹ eine Brücke der Struktur -*O-CH₂-, -*N(CH₃)-CH₂-, -*N(C₂H₅)-CH₂-,-*N(c-C₃H₅)-CH₂- oder - *S-CH₂- bilden kann, wobei das mit * markierte Atom mit dem Kohlenstoffatom von D verknüpft ist, und
T einen Rest der Formel 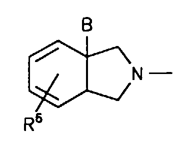
bedeutet, worin
B für -NR³R⁴ oder -OH steht, worin
R³ für Wasserstoff oder Methyl,
R⁴ für Wasserstoff oder Methyl und
R⁶ für Wasserstoff steht
und deren pharmazeutisch verwendbaren Hydrate und Säureadditionssalze sowie die Alkali-, Erdalkali-, Silber- und Guanidiniumsalze der zugrundeliegenden Carbonsäuren.

3. Verbindungen der Formel (I) gemäß Anspruch 1, in welcher
Q einen Rest der Formeln 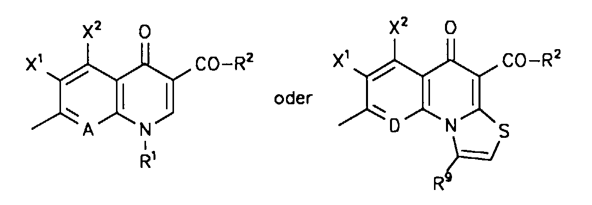
bedeutet, worin
R¹ für gegebenenfalls durch Fluor ein- oder zweifach substituiertes Alkyl mit 1 bis 4 Kohlenstoffatomen, Vinyl, gegebenenfalls durch 1 Fluoratom substituiertes Cyclopropyl, gegebenenfalls durch Fluor ein- oder zweifach substituiertes Phenyl,
R² für Hydroxy, Alkoxy mit 1 bis 2 Kohlenstoffatomen oder (5-Methyl-2-oxo-1,3-dioxol-4-yl)-methoxy,
R⁹ für Wasserstoff oder gegebenenfalls durch Fluor ein- bis dreifach substituiertes Methyl,
X¹ für Fluor,
X² für Wasserstoff, Fluor, Amino, Methyl oder Vinyl,
A für N oder C-R⁷ steht, worin
R⁷ für Wasserstoff, Fluor, Chlor, Brom, CF₃, OCH₃, OCHF₂, CH₃, CN, CH=CH₂ oder C≡CH steht oder auch gemeinsam mit R¹ eine Brücke der Struktur -*O-CH₂-CH-CH₃ oder -*O-CH₂-N-R⁸, wobei das mit * markierte Atom mit dem Kohlenstoffatom von A verknüpft ist und worin
R⁸ Wasserstoff oder Methyl bedeutet, bilden kann, und
D für N oder C-R¹⁰ steht, worin
R¹⁰ für Wasserstoff, Fluor, Chlor oder OCH₃ steht oder auch gemeinsam mit R⁹ eine Brücke der Struktur -*O-CH₂-, -*N(CH₃)-CH₂-, -*N(C₂H₅)-CH₂- oder -*S-CH₂- bilden kann, wobei das mit * markierte Atom mit dem Kohlenstoffatom von D verknüpft ist, und
T einen Rest der Formel 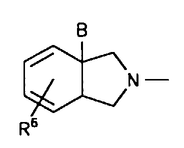
bedeutet, worin
B für NH₂ und
R⁶ für Wasserstoff steht,
und deren pharmazeutisch verwendbaren Hydrate und Säureadditionssalze sowie die Alkali-, Erdalkali-, Silber- und Guanidiniumsalze der zugrundeliegenden Carbonsäuren.

4. Diastereomerenreine und enantiomerenreine Verbindungen nach den Ansprüchen 1 bis 3.

5. Verfahren zur Herstellung von Verbindungen der Formel (I) gemäß Ansprüchen 1 bis 4, dadurch gekennzeichnet, daß Verbindungen der Formel (II)
Y-Q (II),
in welcher
Q die oben angegebene Bedeutung hat und
Y für Halogen, inbesondere für Fluor oder Chlor steht,
mit Verbindungen der Formel (III) 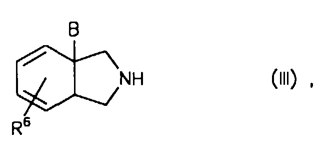 in welcher
B und R⁶ die oben angegebenen Bedeutungen haben,
gegebenenfalls in Gegenwart von Säurefängern umsetzt und gegebenenfalls vorhandene Schutzgruppen abspaltet.

6. Racemische, diastereomerenreine und enantiomerenreine Verbindungen aus der Gruppe bestehend aus
1,2,3,4,7,7a-Hexahydro-isoindol-2,3a-dicarbonsäure-3a-ethylester-2-methylester,
2-Benzyl-1,2,3,4,7,7a-hexahydro-isoindol-3a-carbonsäureethylester, 1,2,3,7a-Tetrahydro-isoindol-2,3a-dicarbonsäure-3a-ethylester-2-methylester, 1,2,3,7a-Tetrahydro-isoindol-2,3a-dicarbonsäure-2-methylester, 3a-Methoxycarbonylamino-1,2,3,7a-tetrahydro-isoindol-2-carbonsäuremethylester,
1,2,3,7a-Tetrahydro-isoindol-3a-ylamin,
4-Methyl-1,2,3,7a-tetrahydro-isoindol-3a-ylamin,
5-Methyl-1,2,3,7a-tetrahydro-isoindol-3a-ylamin,
6-Methyl-1,2,3,7a-tetrahydro-isoindol-3a-ylamin,
7-Methyl-1,2,3,7a-tetrahydro-isoindol-3a-ylamin,
3a-Methylamino-1,2,3,7a-tetrahydro-isoindol,
3a-Dimethylamino-1,2,3,7a-tetrahydro-isoindol,
3a-tert.-Butoxycarbonylamino-1,2,3,7a-tetrahydro-isoindol,
3a-Aminomethyl-1,2,3,7a-tetrahydro-isoindol,
3a-Methylaminomethyl-1,2,3,7a-tetrahydro-isoindol,
3a-Dimethylaminomethyl-1,2,3,7a-tetrahydro-isoindol,
3a-Hydroxy-1,2,3,7a-tetrahydro-isoindol,
3a-Hydroxymethyl-1,2,3,7a-tetrahydro-isoindol.

7. Verbindungen gemäß Ansprüchen 1 bis 4 zur Bekämpfung von Krankheiten.

8. Verbindungen gemäß Ansprüchen 1 bis 4 zur Bekämpfung von bakteriellen Infektionen.

9. Verwendung von Verbindungen gemäß Ansprüchen 1 bis 4 bei der Herstellung von Arzneimitteln.

10. Arzneimittel enthaltend die Verbindungen gemäß Ansprüchen 1 bis 4.

11. Antibakterielle Mittel enthaltend Verbindungen gemäß Ansprüchen 1 bis 4.

## Claims

1. Compounds of the formula (I)
T-Q (I),
in which
Q denotes a radical of the formula 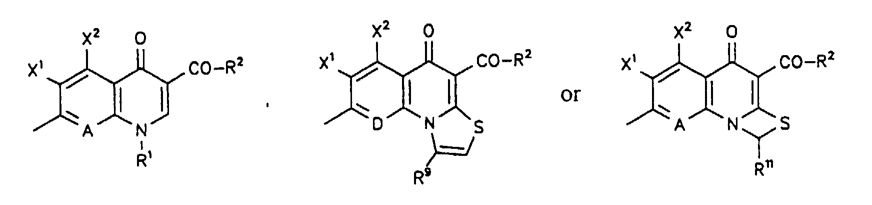 wherein
R¹ represents alkyl having 1 to 4 carbon atoms, which is optionally mono- or disubstituted by halogen or hydroxyl, alkenyl having 2 to 4 carbon atoms, cycloalkyl having 3 to 6 carbon atoms, which is optionally substituted by 1 or 2 fluorine atoms, bicycle[1.1.1]pent-1-yl, 1,1-dimethylpropargyl, 3-oxetanyl, methoxy, amino, methylamino, dimethylamino, or phenyl which is optionally mono- or disubstituted by halogen, amino or hydroxyl,
R² represents hydroxyl, alkoxy having 1 to 3 carbon atoms, which is optionally substituted by hydroxyl, methoxy, amino or dimethylamino, benzyloxy or (5-methyl-2-oxo-1,3-dioxol-4-yl)-methyloxy, acetoxymethyloxy, pivaloyloxymethyloxy, 5-indanyloxy, phthalidinyloxy, 3-acetoxy-2-oxo-butyloxy, nitromethyl or dialkoxycarbonylmethyl having 1 to 2 carbon atoms in each alkyl moiety,
R⁹ represents hydrogen or alkyl having 1 to 3 carbon atoms, which is optionally substituted by methoxy, hydroxy or halogen,
R¹¹ represents hydrogen, CH₃ or CH₂F,
X¹ represents halogen or nitro,
X² represents hydrogen, halogen, amino, hydroxyl, methoxy, mercapto, methyl, halogenomethyl or vinyl,
A represents N or C-R⁷, wherein
R⁷ represents hydrogen, halogen, CF₃, OCH₃, OCHF₂, CH₃, CN, CH=CH₂ or C≡CH or alternatively, together with R', can form a bridge of the structure -*O-CH₂-CH-CH₃, -*S-CH₂-CH₂-, -*S-CH₂-CH-CH₃, -*CH₂-CH₂-CH-CH₃ or -*O-CH₂-N-R⁸, the atom marked by being linked to the carbon atom of A and wherein
R⁸ denotes hydrogen, methyl or formyl,
and
D represents N or C-R¹⁰, wherein
R¹⁰ represents hydrogen, halogen, CF₃, OCH₃, OCHF₂ or CH₃ or alternatively, together with R⁹, can form a bridge of the structure -*O-CH₂-, -*NH-CH₂-, -*N(CH₃)-CH₂-, -*N(C₂H₅)-CH₂-, -*N(c-C₃H₅)-CH₂- or -*S-CH₂-, the atom marked by being linked to the carbon atom of D, and
T denotes a radical of the formula 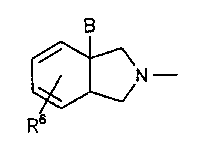 wherein
B represents (CH₂)ₘ-NR³R⁴ or (CH₂)ₘ-OR⁵, wherein
m represents 0 or 1,
R³ represents hydrogen, methyl or alkoxycarbonyl having 1 to 4 carbon atoms in the alkyl moiety,
R⁴ represents hydrogen or methyl and
R⁵ represents hydrogen or methyl and
R⁶ represents hydrogen or methyl,
and their pharmaceutically utilizable hydrates and acid addition salts and also the alkali metal, alkaline earth metal, silver and guanidinium salts of the underlying carboxylic acids.

2. Compounds of the formula (I) according to Claim 1,
in which
Q denotes a radical of the formula 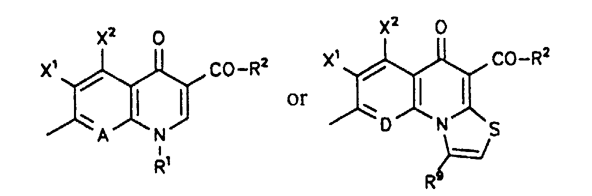 wherein
R¹ represents alkyl having 1 to 4 carbon atoms, which is optionally mono- or disubstituted by halogen, alkenyl having 2 to 3 carbon atoms, cycloalkyl having 3 or 4 carbon atoms, which is optionally substituted by I fluorine atom, bicyclo[1.1.1]pent-1-yl, 1,1-dimethylpropargyl, 3-oxetanyl, methylamino, or phenyl which is optionally mono- or disubstituted by fluorine, amino or hydroxyl,
R² represents hydroxyl, alkoxy having 1 to 2 carbon atoms, benzyloxy or (5-methyl-2-oxo-1,3-dioxol-4-yl)-methoxy,
R⁹ represents hydrogen or alkyl having 1 to 2 carbon atoms, which is optionally mono- to trisubstituted by fluorine,
X¹ represents fluorine or chlorine,
X² represents hydrogen, halogen, amino, methyl, trifluoromethyl or vinyl,
A represents N or C-R⁷, wherein
R⁷ represents hydrogen, halogen, CF₃, OCH₃, OCHF₂, CH₃, CN, CH=CH₂ or C≡CH or alternatively, together with R¹, can form a bridge of the structure -*O-CH₂-CH-CH₃, -*S-CH₂-CH₂-, -*CH₂-CH₂-CH-CH₃ or -*O-CH₂-N-R⁸, the atom marked by * being linked to the carbon atom of A and wherein
R⁸ denotes hydrogen or methyl,
and
D represents N or C-R¹⁰, wherein
R¹⁰ represents hydrogen, fluorine, chlorine, CF₃, OCH₃ or CH₃ or alternatively, together with R⁹, can form a bridge of the structure -*O-CH₂-, -*N(CH₃)-CH₂-, -*N(C₂H₅)-CH₂-, -*N(c-C₃H₅)-CH₂- or -*S-CH₂-, the atom marked by * being linked to the carbon atom of D, and
T denotes a radical of the formula 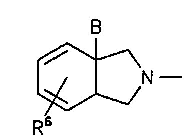 wherein
B represents -NR³R⁴ or -OH, wherein
R³ represents hydrogen or methyl,
R⁴ represents hydrogen or methyl and
R⁶ represents hydrogen
and their pharmaceutically utilizable hydrates and acid addition salts and also the alkali metal, alkaline earth metal, silver and guanidinium salts of the underlying carboxylic acids.

3. Compounds of the formula (I) according to Claim 1, in which
Q denotes a radical of the formula 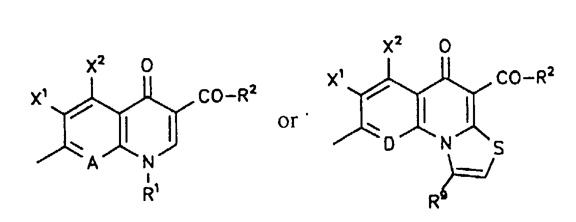 wherein
R¹ represents alkyl having 1 to 4 carbon atoms, which is optionally mono- or disubstituted by fluorine, vinyl, cyclopropyl which is optionally substituted by 1 fluorine atom, or phenyl which is optionally mono- or disubstituted by fluorine,
R² represents hydroxyl, alkoxy having 1 to 2 carbon atoms or (5-methyl-2-oxo-1,3-dioxol-4-yl)-methoxy,
R⁹ represents hydrogen or methyl which is optionally mono- to trisubstituted by fluorine,
X¹ represents fluorine,
X² represents hydrogen, fluorine, amino, methyl or vinyl,
A represents N or C-R⁷, wherein
R⁷ represents hydrogen, fluorine, chlorine, bromine, CF₃, OCH₃, OCHF₂, CH₃, CN, CH=CH₂ or C≡CH or alternatively, together with R¹, can form a bridge of the structure -*O-CH₂-CH-CH₃ or -*O-CH₂-N-R⁸, the atom marked by being linked to the carbon atom of A and wherein
R⁸ denotes hydrogen or methyl,
and
D represents N or C-R¹⁰, wherein
R¹⁰ represents hydrogen, fluorine, chlorine or OCH₃ or alternatively, together with R⁹, can form a bridge of the structure -*O-CH₂-, -*N(CH₃)-CH₂-, -*N(C₂H₅)-CH₂- or -*S-CH₂-, the atom marked by * being linked to the carbon atom of D, and
T denotes a radical of the formula 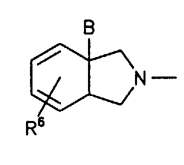 wherein
B represents NH₂ and
R⁶ represents hydrogen,
and their pharmaceutically utilizable hydrates and acid addition salts and also the alkali metal, alkaline earth metal, silver and guanidinium salts of the underlying carboxylic acids.

4. Diasteromerically pure and enantiomerically pure compounds according to Claims 1 to 3.

5. Process for the preparation of compounds of the formula (I) according to Claims 1 to 4, characterized in that compounds of the formula (II)
Y-Q (II),
in which
Q has the meaning indicated above and
Y represents halogen, in particular fluorine or chlorine,
are reacted with compounds of the formula (III) 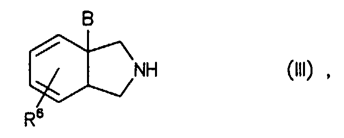 in which
B and R⁶ have the meanings indicated above,
if appropriate in the presence of acid scavengers and, if appropriate, protective groups present are removed.

6. Racemic, diastereomerically pure and enantiomerically pure compounds from the group consisting of
3a-ethyl 2-methyl 1,2,3,4,7,7a-hexahydro-isoindole-2,3a-dicarboxylate,
2-benzyl-1,2,3,4,7,7a-hexahydro-isoindole-3a-carboxylic acid ethyl ester,
3a-ethyl 2-methyl 1,2,3,7a-tetrahydro-isoindole-2,3a-dicarboxylate,
1,2,3,7a-tetrahydro-isoindole-2,3a-dicarboxylic acid 2-methyl ester, methyl 3a-methoxycarbonylamino-1,2,3,7a-tetrahydro-isoindole-2-carboxylate,
1,2,3,7a-tetrahydro-isoindol-3a-ylamine,
4-methyl-1,2,3,7a-tetrahydro-isoindol-3a-ylamine,
5-methyl-1,2,3,7a-tetrahydro-isoindol-3a-ylamine,
6-methyl-1,2,3,7a-tetrahydro-isoindol-3a-ylamine,
7-methyl-1,2,3,7a-tetrahydro-isoindol-3a-ylamine,
3a-methylamino-1,2,3,7a-tetrahydro-isoindole,
3a-dimethylamino-1,2,3,7a-tetrahydro-isoindole,
3a-tert-butoxycarbonylamino-1,2,3,7a-tetrahydro-isoindole,
3a-aminomethyl-1,2,3,7a-tetrahydro-isoindole,
3a-methylaminomethyl-1,2,3,7a-tetrahydro-isoindole,
3a-dimethylaminomethyl-1,2,3,7a-tetrahydro-isoindole,
3a-hydroxy-1,2,3,7a-tetrahydro-isoindole,
3a-hydroxymethyl-1,2,3,7a-tetrahydro-isoindole.

7. Compounds according to Claims 1 to 4 for the control of diseases.

8. Compounds according to Claims 1 to 4 for the control of bacterial infections.

9. Use of compounds according to Claims 1 to 4 in the production of medicaments.

10. Medicaments containing the compounds according to Claims 1 to 4.

11. Antibacterial compositions containing compounds according to Claims 1 to 4.

## Revendications

1. Composés de formule (I)
T-Q (I)
dans laquelle
Q est un reste de formules 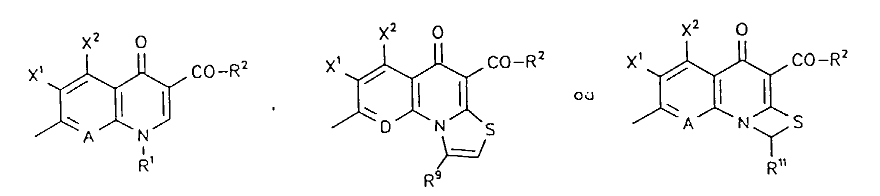 dans lesquelles
R¹ est un groupe alkyle ayant 1 à 4 atomes de carbone éventuellement substitué une ou deux fois par un halogène ou un radical hydroxy, un groupe alcényle ayant 2 à 4 atomes de carbone, un groupe cycloalkyle de 3 à 6 atomes de carbone éventuellement substitué par 1 ou 2 atomes de fluor, un groupe bicyclo[1.1.1]pent-1-yle, 1,1-diméthylpropargyle, 3-oxétanyle, méthoxy, amino, méthylamino, diméthylamino, un groupe phényle éventuellement substitué une ou deux fois par un halogène, un radical amino ou hydroxy,
R² est un groupe hydroxy, un groupe alkoxy ayant 1 à 3 atomes de carbone éventuellement substitué par un radical hydroxy, méthoxy, amino, diméthylamino, un groupe benzyloxy ou un groupe (5-méthyl-2-oxo-1,3-dioxol-4-yl)-méthyloxy, acétoxyméthyloxy, pivaloyloxyméthyloxy, 5-indanyloxy, phtalidinyloxy, 3-acétoxy-2-oxo-butyloxy, nitrométhyle ou un groupe dialkoxycarbonylméthyle ayant 1 ou 2 atomes de carbone dans chaque partie alkyle,
R⁹ représente l'hydrogène ou un groupe alkyle ayant 1 à 3 atomes de carbone éventuellement substitué par un radical méthoxy, hydroxy ou un halogène,
R¹¹ représente l'hydrogène, un groupe CH₃ ou CH₂F,
X¹ est un halogène ou un groupe nitro,
X² représente l'hydrogène, un halogène, un groupe amino, hydroxy, méthoxy, mercapto, méthyle, halogénométhyle ou vinyle,
A représente N ou un groupe C-R⁷, dans lequel
R⁷ est l'hydrogène, un halogène, un radical CF₃, OCH₃, OCHF₂, CH₃, CN, CH=CH₂ ou C≡CH ou peut aussi former conjointement avec R¹ un pont de structure -*O-CH₂-CH-CH₃, -*S-CH₂-CH₂-, -*S-CH₂-CH-CH₃, -*CH₂-CH₂-CH-CH₃ ou -*O-CH₂-N-R⁸, l'atome marqué d'un signe * étant lié à l'atome de carbone de A et
R⁸ représente l'hydrogène, un groupe méthyle ou formyle,
et
D représente N ou un groupe C-R¹⁰ dans lequel
R¹⁰ est l'hydrogène, un halogène, un radical CF₃, OCH₃, OCHF₂ ou CH₃ ou peut former conjointement avec R⁹ un pont de structure -*O-CH₂-, -*NH-CH₂-, -*N(CH₃)-CH₂-, -*N(C₂H₅)-CH₂-, -*N(c-C₃H₅)-CH₂- ou -*S-CH₂-, l'atome marqué d'un signe * étant lié à l'atome de carbone de D, et
T représente un reste de formule 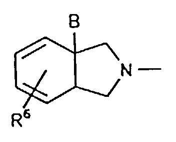 dans laquelle
B est un groupe (CH₂)ₘ-NR³R⁴ ou (CH₂)ₘ-OR⁵, où
m a la valeur 0 ou 1,
R³ est l'hydrogène, un groupe méthyle ou un groupe alkoxycarbonyle ayant 1 à 4 atomes de carbone dans la partie alkyle,
R⁴ représente l'hydrogène ou un groupe méthyle et
R⁵ représente l'hydrogène ou un groupe méthyle et
R⁶ est l'hydrogène ou un groupe méthyle,
et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

2. Composés de formule (I) suivant la revendication 1,
dans laquelle
Q représente un reste de formules 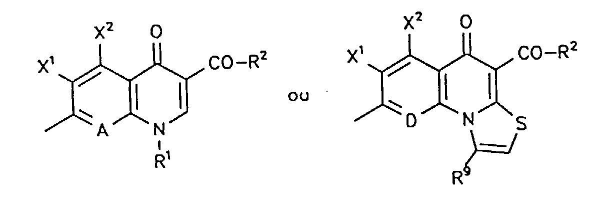 dans lesquelles
R¹ est un groupe alkyle ayant 1 à 4 atomes de carbone éventuellement substitué une ou deux fois par un halogène, un groupe alcényle ayant 2 ou 3 atomes de carbone, un groupe cycloalkyle de 3 ou 4 atomes de carbone éventuellement substitué par 1 atome de fluor, un groupe bicyclo[1.1.1]pent-1-yle, 1,1-diméthylpropargyle, 3-oxétanyle, méthylamino, un groupe phényle éventuellement substitué une ou deux fois par du fluor, un radical amino ou hydroxy,
R² est un groupe hydroxy, un groupe alkoxy ayant 1 ou 2 atomes de carbone, un groupe benzyloxy ou (5-méthyl-2-oxo-1,3-dioxol-4-yl)-méthoxy,
R⁹ représente l'hydrogène ou un groupe alkyle ayant 1 ou 2 atomes de carbone éventuellement substitué une à trois fois par du fluor,
X¹ représente le fluor ou le chlore,
X² est l'hydrogène, un halogène, un groupe amino, méthyle, trifluorométhyle ou vinyle,
A représente N ou un groupe C-R⁷ dans lequel
R⁷ est l'hydrogène, un halogène, un radical CF₃, OCH₃, OCHF₂, CH₃, CN, CH=CH₂ ou C≡CH ou peut aussi former conjointement avec R¹ un pont de structure -*O-CH₂-CH-CH₃, -*S-CH₂-CH₂-, -*CH₂-CH₂-CH-CH₃ ou -*O-CH₂-N-R⁸, où l'atome marqué d'un signe * est lié à l'atome de carbone de A et où
R⁸ représente l'hydrogène ou un groupe méthyle,
et
D représente N ou un groupe C-R¹⁰ dans lequel
R¹⁰ est l'hydrogène, le fluor, le chlore, un radical CF₃, OCH₃ ou CH₃ ou peut aussi former conjointement avec R⁹ un pont de structure -*O-CH₂-, -*N(CH₃)-CH₂-, -*N(C₂H₅)-CH₂-, -*N(c-C₃H₅)-CH₂- ou -*S-CH₂-, l'atome marqué d'un signe * étant lié à l'atome de carbone de D, et
T représente un reste de formule 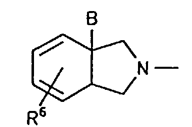 dans laquelle
B est un groupe -NR³R⁴ ou -OH, où
R³ est l'hydrogène ou un groupe méthyle,
R⁴ est l'hydrogène ou un groupe méthyle et
R⁶ est l'hydrogène,
et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

3. Composés de formule (I) suivant la revendication 1, dans laquelle
Q représente un reste de formules 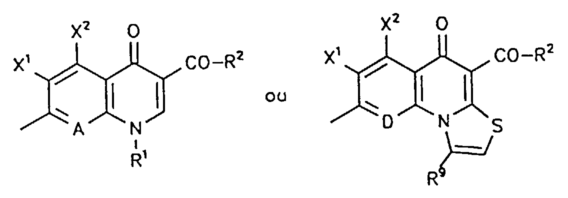 où
R¹ est un groupe alkyle ayant 1 à 4 atomes de carbone éventuellement substitué une ou deux fois par du fluor, un groupe vinyle, un groupe cyclopropyle éventuellement substitué par un atome de fluor, un groupe phényle éventuellement substitué une ou deux fois par du fluor,
R² est un groupe hydroxy, un groupe alkoxy ayant 1 ou 2 atomes de carbone ou un groupe (5-méthyl-2-oxo-1,3-dioxol-4-yl)-méthoxy,
R⁹ représente l'hydrogène ou un groupe méthyle éventuellement substitué une à trois fois par du fluor,
X¹ représente le fluor,
X² représente l'hydrogène, le fluor, un groupe amino, méthyle ou vinyle,
A représente N ou un groupe C-R⁷, dans lequel
R⁷ est l'hydrogène, le fluor, le chlore, le brome, un radical CF₃, OCH₃, OCHF₂, CH₃, CN, CH=CH₂ ou C≡CH ou peut aussi former conjointement avec R¹ un pont de structure -*O-CH₂-CH-CH₃ ou -*O-CH₂-N-R⁸, dans lequel l'atome marqué d'un signe * est lié à l'atome de carbone de A et où
R⁸ représente l'hydrogène ou un groupe méthyle,
et
D représente N ou un groupe C-R¹⁰ dans lequel
R¹⁰ est l'hydrogène, le fluor, le chlore ou un radical OCH₃ ou peut aussi former conjointement avec R⁹ un pont de structure -*O-CH₂-, -*N(CH₃)-CH₂-, -*N(C₂H₅)-CH₂- ou -*S-CH₂-, l'atome marqué d'un signe * étant lié à l'atome de carbone de D, et
T représente un reste de formule 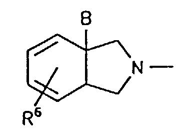 dans laquelle
B est un groupe NH₂ et
R⁶ est l'hydrogène,
et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

4. Composés diastéréo-isomères purs et énantiomères purs suivant les revendications 1 à 3.

5. Procédé de production de composés de formule (I) suivant les revendications 1 à 4, caractérisé en ce qu'on fait réagir des composés de formule (II)
Y-Q (II)
dans laquelle
Q a la définition indiquée ci-dessus et
Y représente un halogène, en particulier le fluor ou le chlore,
avec des composés de formule (III) 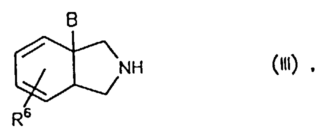 dans laquelle
B et R⁶ ont les définitions indiquées ci-dessus,
le cas échéant en présence d'accepteurs d'acides et on élimine les groupes protecteurs éventuellement présents.

6. Composés racémiques, diastéréo-isomères purs et énantiomères purs du groupe consistant en
l'ester 3a-éthylique et 2-méthylique de l'acide 1,2,3,4,7,7a-hexahydro-iso-indole-2,3a-dicarboxylique,
l'ester éthylique de l'acide 2-benzyl-1,2,3,4,7,7a-hexahydro-iso-indole-3a-carboxylique,
l'ester 3a-éthylique et 2-méthylique de l'acide 1,2,3,7a-tétrahydro-iso-indole-2,3a-dicarboxylique,
l'ester 2-méthylique de l'acide 1,2,3,7a-tétrahydro-iso-indole-2,3a-dicarboxylique,
l'ester méthylique de l'acide 3a-méthoxycarbonylamino-1,2,3,7a-tétrahydro-iso-indole-2-carboxylique,
la 1,2,3,7a-tétrahydro-iso-indole-3a-ylamine,
la 4-méthyl-1,2,3,7a-tétrahydro-iso-indole-3a-ylamine,
la 5-méthyl-1,2,3,7a-tétrahydro-iso-indole-3a-ylamine,
la 6-méthyl-1,2,3,7a-tétrahydro-iso-indole-3a-ylamine,
la 7-méthyl-1,2,3,7a-tétrahydro-iso-indole-3a-ylamine,
le 3a-méthylamino-1,2,3,7a-tétrahydro-iso-indole,
le 3a-diméthylamino-1,2,3,7a-tétrahydro-iso-indole,
le 3a-tertio-butoxycarbonylamino-1,2,3,7a-tétrahydro-iso-indole,
le 3a-aminométhyl-1,2,3,7a-tétrahydro-iso-indole,
le 3a-méthylaminométhyl-1,2,3,7a-tétrahydro-iso-indole,
le 3a-diméthylaminométhyl-1,2,3,7a-tétrahydro-iso-indole,
le 3a-hydroxy-1,2,3,7a-tétrahydro-iso-indole,
le 3a-hydroxyméthyl-1,2,3,7a-tétrahydro-iso-indole.

7. Composés suivant les revendications 1 à 4, destinés à être utilisés pour combattre des maladies.

8. Composés suivant les revendications 1 à 4, destinés à être utilisés pour combattre des infections bactériennes.

9. Utilisation de composés suivant les revendications 1 à 4 dans la préparation de médicaments.

10. Médicaments contenant les composés suivant les revendications 1 à 4.

11. Composition antibactérienne contenant des composés suivant les revendications 1 à 4.
